# EUROPEAN PATENT APPLICATION

(11) **EP 2 107 125 A1**
(43) Date of publication of application: **07.10.2009**
(21) Application number: 08006466.0
(22) Date of filing: 31.03.2008
(51) Int. Cl.: C12Q 1/68, B01L 7/00, B01L 3/00

(54) **Real-time PCR of targets on a micro-array**

(71) Applicant: Eppendorf Array Technologies SA (EAT), 5000 Namur (BE)
(72) Inventor: Remacle, José, 5020 Malonne (BE); Alexandre, Isabelle, 5000 Namur (BE); De Roeck, Sven, 1050 Bruxelles (BE); Husar, Dieter, 22367 Hamburg (DE); Zammatteo, Nathalie, 5000 Namur (BE); Koehn, Heinz, 22339 Hamburg (DE)
(74) Representative: Becker Kurig Straus

(57) **Abstract**

The present invention relates to a method, apparatus, cartridge and kit for monitoring on a micro-array a real-time PCR amplification of a polynucleotide molecule being present in a solution.

## Description

### Field of the invention

The present invention relates to a method and an apparatus for detection of multiple targets being possibly present in a sample in conjunction with their amplification by PCR or even in Real Time PCR. The invention is based on performing a PCR for nucleic acid amplification over multiple thermal cycles in a chamber containing capture probes immobilized on a flat surface for capturing the amplicons formed during the PCR. More particularly, the invention allows the detection and quantification of polynucleotide molecule in a real time PCR amplification using micro-arrays. The invention also comprises means and apparatus for performing the method.

### Description of the related art

The disclosed polynucleotide molecule amplification method offers the advantages of making an efficient amplification in a chamber that is compatible with the detection of the amplified molecules on immobilized capture probes in the same chamber. Nucleic acid amplification and detection are very useful in many applications such as medical diagnostic assays.

The sensitivity and specificity of nucleic acid detection methods were greatly improved by the invention of the polymerase chain reaction (PCR). PCR is a process for amplifying nucleic acids and involves the use of two oligonucleotide primers, an agent for polymerization, a target nucleic acid template, and successive cycles of denaturation of nucleic acid and annealing and extension of the primers to produce a large number of copies of a particular nucleic acid segment. With this method, segments of single copy genomic DNA are amplified with very high specificity and fidelity and the extend of the amplification can be as high as several million fold.

Methods for detecting PCR products are well described in U.S. Pat. No. 4,683,195. To be specific, the detection requires the use of a polynucleotide probe capable of hybridizing specifically with the amplified target nucleic acid. These methods require separate steps of amplification, capture, and detection and generally require several hours to be complete.

Due to the enormous amplification of the PCR process, small levels of DNA carryover from samples with high DNA content, positive control templates, or from previous amplifications may result in the formation of PCR product even in the absence of purposefully added template DNA. Because the possibility of introducing contaminating DNA to a sample increases with the number of handling steps required for sample preparation, processing, and analysis is increased, it would be preferable to minimize sample handling for their amplification, detection and quantification, particularly when high amount of amplicons are present meaning after the amplification reaction is complete.

Methods for simultaneous amplification and detection of target nucleic acids have been described in order to minimize the problems of sample contamination. The U.S. Pat. No. 4,683,195 and U.S. Pat. No. 6,171,785 involve the introduction of detectable DNA binding agents (such as ethidium bromide) into the amplification reaction, which agents produce a detectable signal that is enhanced upon binding double-stranded DNA. An increased in fluorescence of the PCR mixture indicates that amplification has occurred. The U.S. Pat. No. 6,395,518 and U.S. Pat. No. 5,952,202 discloses an oligonucleotide probe including a fluorescer molecule attached to a first end of the oligonucleotide and a quencher molecule attached to the opposite end of the oligonucleotide such that the fluorescer is substantially unquenched whenever the oligonucleotide probe is in a double-stranded state. A DNA polymerase having 5' to 3' nuclease activity digests said probes during amplification to separate the reporter dye from the quencher. An increased in fluorescence of the PCR mixture indicates that amplification has occurred. The U.S. Pat. No. 5,716,784 provides an alternative method based on the use of two complementary probes, the first analytical probe being labelled at its 5' terminus with an energy transfer donor fluorophore, and the second detection probe being labelled at its 3' terminus with an energy transfer acceptor fluorophore. Measurement of oligonucleotide analytical probe hybridized in solution to oligonucleotide detection probe measured spectrophotometrically in solution by energy transfer measurement, provides a measure of the amount of oligonucleotide analytical probe used up in the amplification of the target nucleic acid sequence and thus provides a measure of amount of target nucleic acid sequence amplified in the PCR replication procedure. The U.S. Pat. No. 5,928,907 describes an apparatus for monitoring the formation of a nucleic acid amplification reaction product in real time which uses a fiber optic focused in the volume of the sample.

Although those methods are capable of monitoring in real time the quantification of nucleic acids in a homogeneous PCR hybridization system, they do it in solution and are limited to the quantification of one target nucleic acid per fluorescent dye. The multiplexing is not easy to implement due to the requirement of non overlapping fluorescent dyes for measuring the increase in signal related to the amplification of several target nucleic acids in the same apparatus.

One solution to the problem was provided by the WO06053770A1 which proposed to amplify the targets polynucleotide sequence in the presence of multiple immobilized capture molecules for their detection. However the implementation of this invention leads to constraints on the efficiency of PCR which has to be compatible with the detection of the targets bound to the capture probes present on a support. Also one of the difficulty of these combined methods is the formation of bubbles during the PCR.

There have been a number of means developed for minimizing the adverse impact of bubble formation. For example, a surfactant may be added to the hybridization solution and the solution continuously mixed to ensure that the bubbles are mobile and prevented from remaining at any particular location. In addition, U.S. Pat. No. 5,959,098 to Goldberg et al., U.S. Pat. No. 5,945,334 to Besemer et al., U.S. Pat. No. 5,922,591 to Anderson et al. and U.S. Pat. No. 6,458,526 Schembri et al. each describe other bubble management means such as employing nonparallel top and bottom surfaces in a hybridization chamber to reduce the potential of trapping the bubbles. Another such bubble management means involves moving the hybridization solution in and out of the chamber throughout the hybridization process. Another bubble inhibition means involves a reaction chamber comprising a base and a cover subtantially parallel creating a gas-fluid interface having a gaz fluid radius that is selected to provide a predetermined radius below which a bubble will shrink. All these references address the problems associated with bubble formation or their inhibition. It has been recently showed that air bubble formation during PCR in microreactors has been reported as one of the major causes of PCR failure in such microdevices (Liu et al., 2007, J. Micromech.Microeng. 17, 2055-2064).

### Summary of the invention

The present invention aims to propose a method and apparatus for the simultaneous amplification of multiple target molecules in a chamber where they can be detected simultaneously on immobilized probes.

Technically, the present invention provides a simple solution to a difficult problem which is the possibility to amplify multiple target molecules with a high efficiency in a chamber having a large flat and transparent surface and keep these features throughout the PCR cycles so that in the process of the PCR or at the end of the amplification the surface is perfectly homogeneous and flat for the detection and quantification of the immobilized target molecules. The same chamber and the same solution are preferably used during the amplification in order to detect through the support the targets bound to their respective capture molecules at specific localized area on the surface of the support so that both PCR and detections are performed in a single process allowing real time PCR simultaneously on multiple targets.

The present invention gives a solution to the possibility to detect the bound target on the surface during the amplification which can be performed without detaching the chamber from the holder on which it is fixed for the PCR.

In order to realize the above-mentioned objectives, the method for PCR amplification and detection of a polynucleotide molecule being present in a solution contained in a chamber having a flat surface bearing the capture molecules for the detection of the amplified sequences comprises the steps of:
- providing a rotating holder (1) and a reaction chamber (2) having fixed upon one of its surface at least a capture molecule (20) being immobilized in a localized areas (21) of a flat surface of said reaction chamber,
- introducing a solution containing said polynucleotide molecule into said reaction chamber (2) and reagents for polynucleotide molecule amplification and labelling,
- submitting the solution to at least 2 thermal cycles having at least 2 and preferably 3 different temperature steps in order to obtain labelled target polynucleotide molecule by PCR amplification, wherein the change in temperatures is obtained by changing the temperature of the air around the reaction chamber (2) and wherein the reaction chamber (2) is subjected to a rotating movement,
- performing at least a measurement of the labelled target polynucleotide molecule in the following way,
   - incubating said labelled target polynucleotide molecule present in said solution under conditions allowing a specific hybridization between said target polynucleotide molecule and its corresponding capture molecule (20),
   - measuring a signal originating from the surface having the bound labelled target polynucleotide molecule in response to illumination of said flat surface, wherein the signal is measured outside the chamber, and wherein the surface of signal emission comprises at least one localized area having a surface of between about 0.1 µm² and about 75 mm²,
   wherein the said surface has a homogeneous interface during the measurement of the signal and
- Processing the data obtained in order to detect and/or quantify the amount of polynucleotide molecule present in the solution before the amplification.

The invention further relates to an apparatus for monitoring on a micro-array a PCR amplification of a polynucleotide molecule being present in a solution, said apparatus comprising:
- a rotating holder (1) and a reaction chamber (2) having fixed upon its surface a micro-array, comprising at least one capture molecule (20) being immobilized in specifically localized areas (21) of a flat surface of said chamber, which is in fluid communication in a chamber with said polynucleotide molecule and reagents for polynucleotide molecule amplification and labelling,
- a thermal cycler for carrying out an automated PCR process, said thermal cycler capable of changing the temperature of the air around the chamber for producing labelled target polynucleotide molecule,
- a rotor for rotating said holder (1) and said reaction chamber (2),
- an illumination light source,
- a detector (9) for measuring a signal from the bound labelled target polynucleotide molecule, with said solution being present in the chamber and containing the labelled target polynucleotide molecule wherein the surface of emission for a localized area is comprised between about 0.1 µm² and about 75 mm² and has a homogeneous interface,
wherein the different parts are integrated into the same apparatus in order to read the signal of the bound labelled target polynucleotide molecule during the PCR amplification.

In a specific embodiment a cartridge is provided for monitoring on a micro-array a PCR amplification of a polynucleotide molecule being present in a solution, said cartridge comprising:
- a substrate (optic bloc) comprising a first flat surface and a flat second surface, said first flat surface comprising a micro-array comprising at least 20 different capture molecules (20) being immobilized in specifically localized areas (21); and
- an airlock comprising an inlet port, a mounting surface and a reaction chamber (2); said reaction chamber comprising a channel constructed to permit fluid flow from said inlet port into said reaction chamber (2), wherein said first flat surface of said substrate is mounted with respect to said mounting surface thereby covering said reaction chamber and whereby said micro-array is located inside said reaction chamber (2).

The invention also related to a kit for monitoring on a micro-array a PCR amplification of a polynucleotide molecule being present in a solution, said kit comprising a cartridge having a substrate (optic bloc) comprising a first flat surface and a flat second surface, said first flat surface comprising a micro-array comprising at least 20 different capture molecules (20) being immobilized in specifically localized areas (21) and primers for the amplification of target, wherein the Tm of the immobilized probes for said target is at least 4°C and preferably 6°C higher than the Tm of the two primers specific of said target and a solution for the PCR including glutamate.

### Description of the drawings

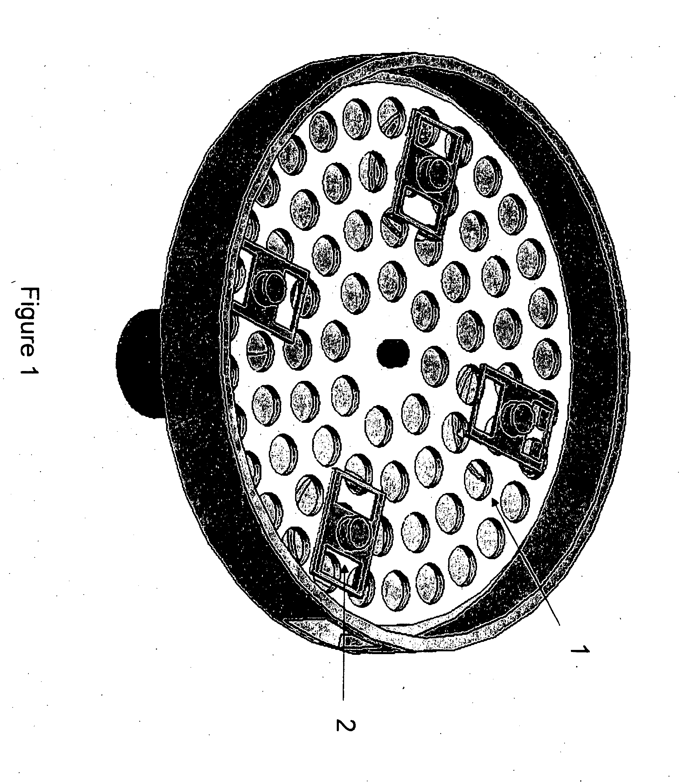

Figure 1. Top view of a schematic presentation of the rotating holder (1) which is fixed on a rotating axis, four closed devices, each device having two reaction chambers (2), one of them having a flat surface with bound capture molecules.

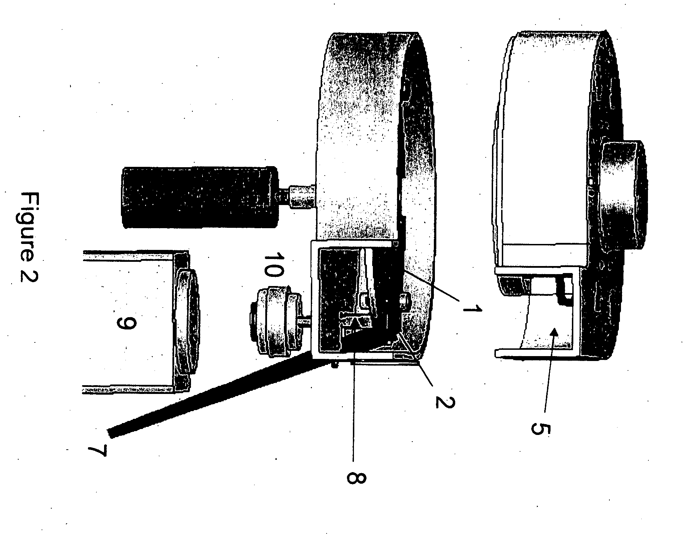

Figure 2. Side view of a general scheme of an integrated apparatus with direct illumination of the bound target and direct detection of the excitation light. The apparatus comprises a rotating holder (1) having a disk shape which is fixed on a rotating axis, a reaction chamber (2) having bound upon a flat surface capture molecules, temperature regulating device (5) based on hot pulsed air (located above the rotating holder), excitation light (7) directed to the surface of the reaction chamber, emission light (8) in the same direction as the excitation light, an optical lens (10) and a detector (9). The illumination and detection systems are located below the rotating holder.

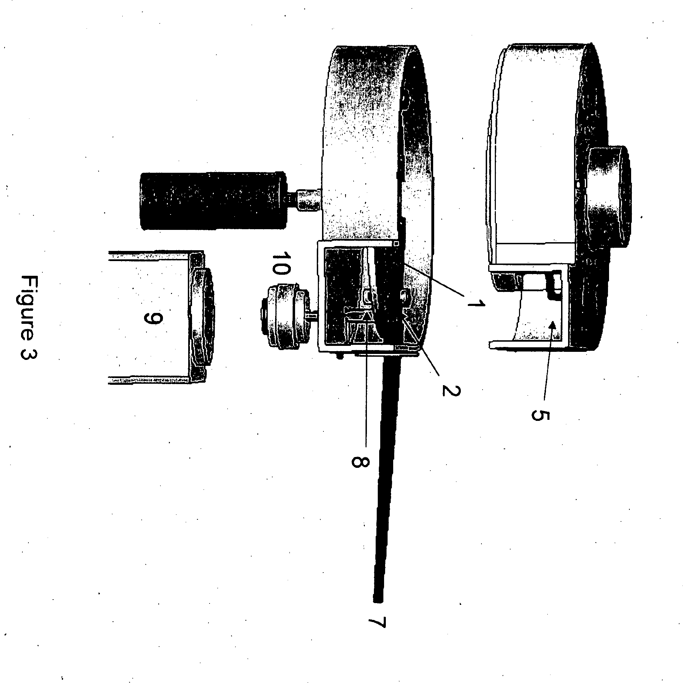

Figure 3. Side view of a general scheme of an integrated apparatus using excitation evanescence. The apparatus comprises a rotating holder (1) having a disk shape which is fixed on a rotating axis, a reaction chamber (2) having bound upon a flat surface capture molecules, temperature regulating device (5) based on hot pulsed air, excitation light (7) producing an evanescent wave, an optical lens (10) and a detector (9). The flat surface of the reaction chamber (2) has a first refractive index (n₁) being higher than the refractive index (n₂) of the solution containing the labelled amplicons. The flat surface of the reaction chamber (2) is illuminated by an excitation light (7), preferably a diverging laser beam or light emitting diode. The emitted light (8) is collected. The bottom of the rotor room comprises a window transparent to the desired wavelength of emitted light (e.g., optically clear or filtered) to allow the reading of the emitted light by the detector (9).

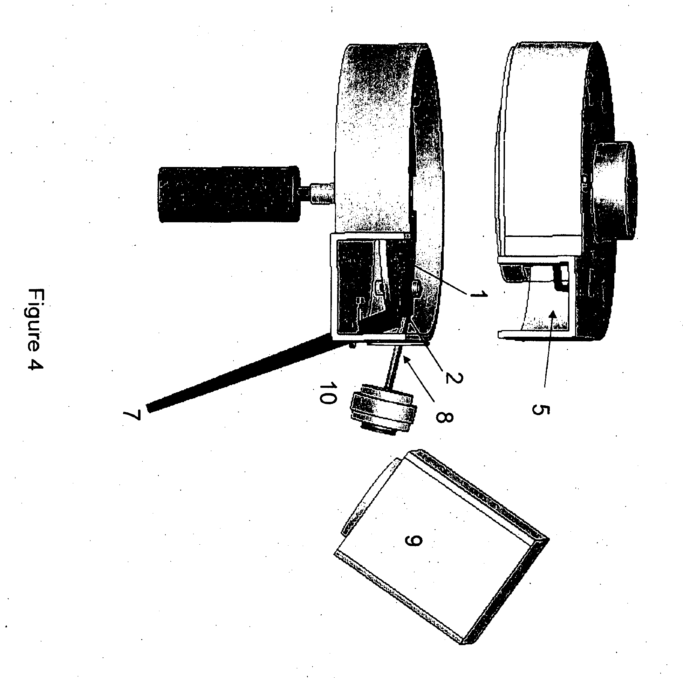

Figure 4. Side view of a general scheme of a preferred integrated apparatus using a detector located in a forbidden angle. The apparatus comprises the rotating holder (1) having a disk shape, a reaction chamber (2) having fixed upon a flat surface capture molecules, temperature regulating device (5) based on hot pulsed air, excitation light (7), an emission light (8), an optical lens (10) and a detector (9) which is localized at a specific observation angle θobin being within the forbidden angle.

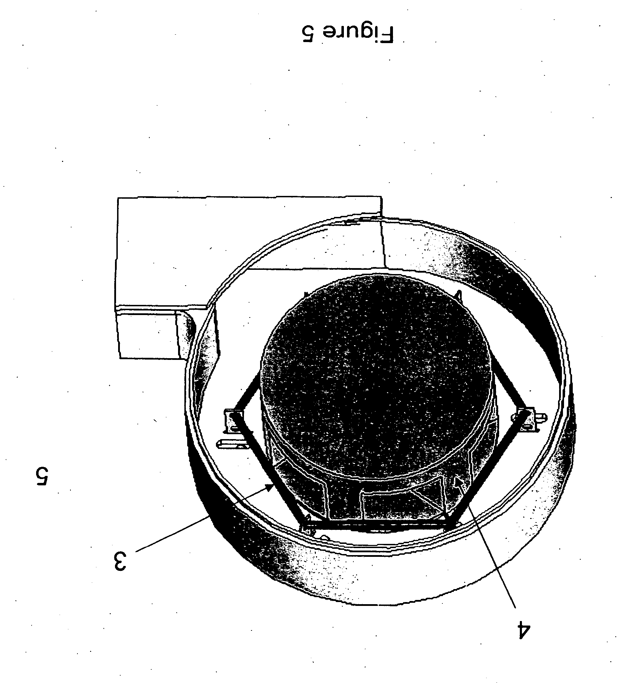

Figure 5. Representation of a preferred temperature regulating device (5) of the invention based on hot pulsed air. The heating system is composed of resistances (3) and vertical fan (4) which are positioned in a cover adapted to be fixed on the rotor room comprising the rotating holder (1) carrying the flat surface devices. The cooling is performed by entering air at room temperature from the outside of the rotor room.

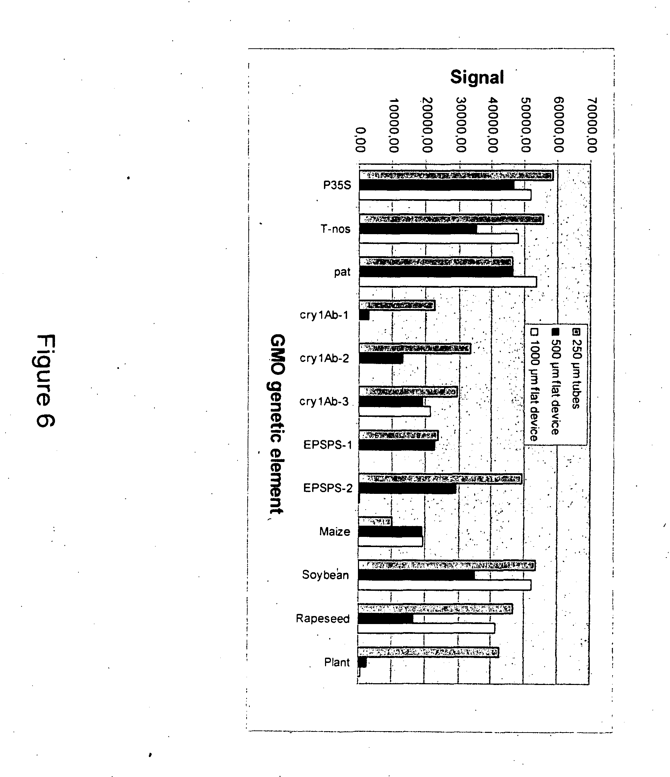

Figure 6. Comparative multiplex PCR amplification on a flat surface or in a PCR tube. Twelve targets were amplified by 40 PCR cycles within a reaction chamber having a flat surface of 4 cm² and being on a support of 500 µm ( dark) or 1000 µm thick( white) or in a PCR tube of 250 µm tube wall( grey). The different amplicons were hybridized on their specific capture probes present on a micro-array in the same solution as used in the PCR as described in the example 1.

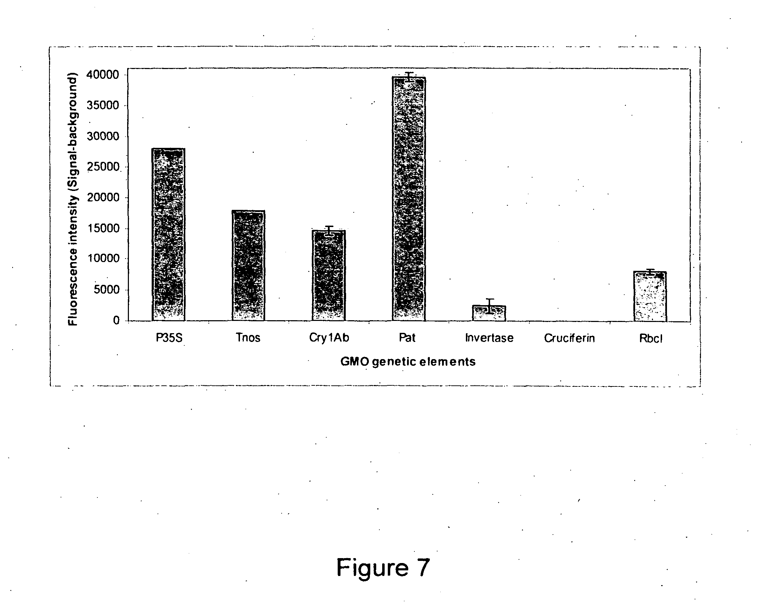

Figure 7. PCR amplification and hybridization on a microarray in a closed device of the invention. The multiplex PCR and hybridiation were performed within a reaction chamber having a flat surface of 40 mm² and being on a support having a thickness of 900 µm. The flat surface comprises a microarray allowing the detection of genetically modified organisms. The sample amplified was 0.1 % Bt11 genomic DNA. This DNA contains 6 genetic elements: P35S, T-nos, Pat, Cry1Ab, Invertase and Rbc1. The 40 PCR cycles using such a heating device were immediately followed by 15 min hybridization on the array present in the same closed chamber. The fluorescent signals on the array were measured with a fluorescent scanner. Details of the experiment are given in example 3.

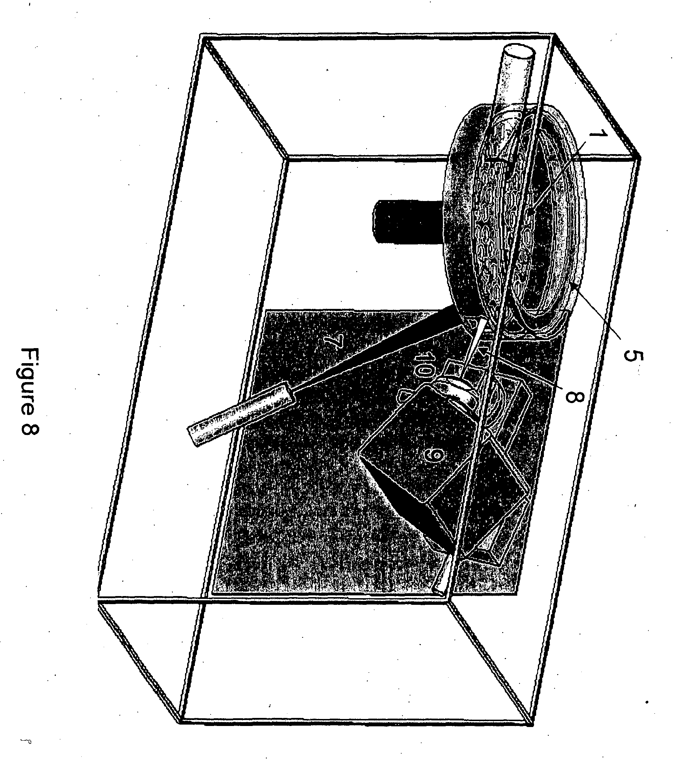

Figure 8. Schematic representation of a preferred integrated apparatus showing the different main components required for the invention: a rotor room comprising the rotating holder (1) having a disk shape which is fixed on a rotating axis and a temperature regulating device (5), excitation light (7), emission light (8), lens (10) and detector (9). The rotor room is isolated from the illumination and detection systems.

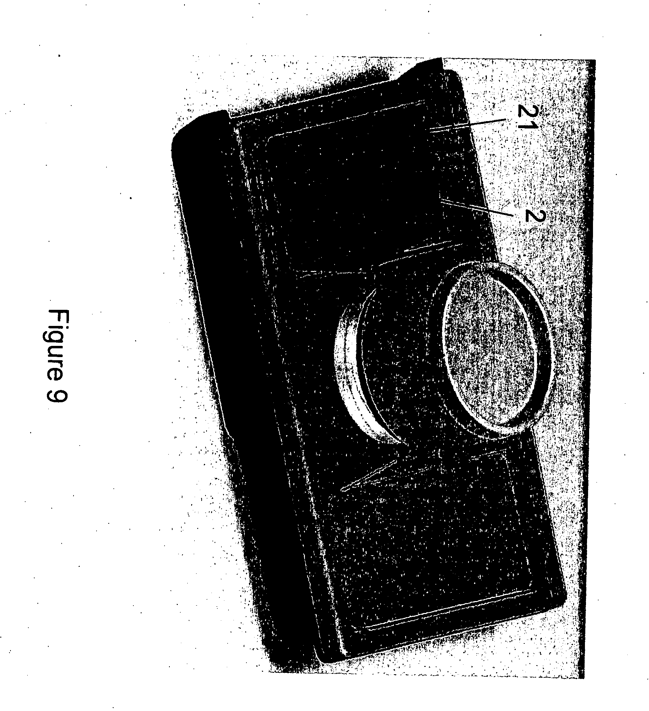

Figure 9. Schematic representation of a preferred cartridge of the invention. The cartridge is preferably composed of two connected reaction chambers, one being the reaction chamber comprising the micro-array where the detection is performed and a second chamber.

The cartridge comprises:
- a substrate comprising a first flat surface and a flat second surface, said first flat surface having fixed upon one of its surface at least a capture molecule (20) being immobilized in a localized areas (21) in the form of a micro-array;
- an airlock comprising an inlet port (screwing cap);
- a mounting surface and a reaction chamber (2), said reaction chamber comprising a channel constructed to permit fluid flow from said inlet port into said reaction chamber (2), whereby said first flat surface of said substrate is mounted with respect to said mounting surface thereby covering said reaction chamber and whereby said micro-array is located inside said reaction chamber (2).
- a second reaction chamber having a channel constructed to permit fluid flow from said inlet port into said second reaction chamber, said channel being connected to the channel constructed to permit fluid flow from said inlet port into the reaction chamber (2) carrying the micro-array.

### Detailed description of the invention

### Definitions

In the context of the present application and invention the following definitions apply:
As used herein, "capture molecule" refers to a molecule, or complex or combination thereof, that is capable of specifically binding to one target molecule, or to a family of target molecules, or to one or more member (s) of a plurality of target molecules, or portion(s) thereof. The capture molecules are preferably nucleic acids, which are either synthesized chemically in situ on the surface of the support or laid down thereon. Nucleic acid binding is achieved via base pairing between two oligonucleotides, one being the immobilized capture molecule and the other one the target to be detected. Capture molecule also comprises derivative of the nucleic acid such as PNA or LNA as long as they can bind specifically the target polynucleotide molecule.

The term "single capture probe species" is a composition of related polynucleotides for the detection of a given sequence by base pairing hybridization or by molecular recognition between polypeptides or proteins. Polynucleotides are synthesized either chemically or enzymatically or purified from samples but the synthesis or purification is not always perfect and the capture molecule is contaminated by other related molecules like shorter polynucleotides. The essential characteristic of one capture species for the invention is that the overall species can be used for capture of a given target polynucleotide molecule.

The term "directly on the surface of the support" means that the main part of the light beam is directed on the surface of the support and excites itself the fluorescence molecules being present on the surface. The term directly differentiate from for example the evanescence excitation which is considered as indirect illumination.

The terms "nucleic acid, micro-array, probe, target nucleic acid, bind substantially, hybridizing specifically to, background, quantifying" are as described in the international patent application WO97/27317, which is incorporated herein by way of reference.

The term "polynucleotide triphosphate" also called dNTP refers to polynucleotides present in either as DNA or RNA and thus includes polynucleotides, which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "polynucleotide" as used herein refers to nucleosides present in nucleic acids (either DNA or RNA) compared with the bases of said nucleic acid, and includes polynucleotides comprising usual or modified bases as above described.

References to polynucleotide(s), polynucleotide(s) and the like include analogous species
wherein the sugar-phosphate backbone is modified and/or replaced, provided that its hybridization properties are not destroyed. By way of example the backbone may be replaced by an equivalent synthetic peptide, called Peptide Nucleic Acid (PNA).

The term "polynucleotide" sequences that are complementary to one or more genes or to the genome sequence described herein, refers to polynucleotides that are capable of hybridizing under stringent conditions to at least part of the polynucleotide sequence of said genes or genome or copy thereof. Polynucleotides also include oligonucleotides being of more than 2 bases but below 100 bases long which can be used under particular conditions. Such hybridizable polynucleotides will typically exhibit at least about 75% sequence identity at the polynucleotide level to said genes or genome, preferably about 80% or 95% sequence identity or preferably more than 95% polynucleotide sequence identity to said genes or genome. They are composed of either small sequences typically 15-30 base long or longer ones being between 30 and 100 or even longer between 100 and 800 base long depending on the specificity and sensitivity requirements for the assay.

The term "homology" is intended to mean the degree of identity of one polynucleotide sequence to another polynucleotide sequence. There may be complete homology (i.e. 100% identity) between two or more polynucleotides. The degree of homology is calculated after alignment of the sequence and may be determined by any methods well known for a person skilled in the art. "Micro-array" means a support on which multiple capture molecules are immobilized in order to be able to bind to the given specific target molecule. The micro-array is preferentially composed of capture molecules present at specifically localized areas on the surface or within the support or on the substrate covering the support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and seen by the detector. A spot is the area where specific target molecules are fixed on their capture molecules and seen by the detector. In one particular application of this invention, micro-arrays of capture molecules are also provided on different supports as long as the different supports contain specific capture molecules and may be distinguished from each other in order to be able to quantify the specific target molecules. This can be achieved by using a mixture of beads having particular features and being able to be recognized from each other in order to quantify the bound molecules. One bead or a population of beads is then considered as a spot having a capture molecule specific of one target molecule.

The terms "background" or "background signal intensity" refers to hybridization signals resulting from non-specific binding, or other non specific interactions, between the labelled target nucleic acids and components of the polynucleotide micro-array (e. g. the polynucleotide probes, control probes, the micro-array substrate, etc.). Background signals may also be produced by intrinsic fluorescence of the micro-array components themselves. A single background signal can be calculated for the entire micro-array, or different background signals may be calculated for each target nucleic acid. In a preferred embodiment, the background is calculated individually for each spot, being the level intensity of the signal on the surface surrounding the spot and not bearing the specific capture molecule.

The polynucleotide molecules of the invention are typically detected by detecting one or more "labels" attached to the polynucleotide molecule. The labels may be incorporated by any of a number of means well known to those of skill in the art, such as detailed in WO 99/32660, which is incorporated herein by way of reference. The label is detected directly preferably in fluorescence.

The polynucleotide molecule is intended to mean a polynucleotide present in the biological material of interest and to be detected. They are obtained either after extraction or purification of the molecules of interest present in a sample being preferentially a biological material. The term "biological material" includes within its meaning organisms, organs, tissues, cells or biological material produced by a cell culture.

The term "Bubble" refers to a small volume of gas in a fluid. The word "bubble" used alone encompasses both a gas bubble and a vapour bubble. The presence of air bubbles in the liquid renders the solution non homogeneous. Similarly the presence of bubbles on a surface makes the surface non homogeneous with its surrounding being either air or a solution.

The term "flat surface" means that the surface is maintained flat at temperatures higher than 85°C, preferably higher than 95°C. The hybridized amplicons have to be detected by a detector and preferably the different localized area that contain specific capture molecules have to show similar signal intensity (with a CV ranging from 0 to 50%) if bound with the same amount of targets. In a preferred embodiment, the flatness tolerance of this surface is less than about 800 microns, preferably less than about 400 microns and even more preferably less than about 100 microns. Preferably, the flatness tolerance of different detected localized areas is less than 100 microns, preferably less than 25 microns.

The term "hot start PCR system" means a composition to allow the DNA polymerase to be active or fully active only after a first incubation at high temperature. The "hot start PCR system" reduces or avoids primer-dimer formation and non specific amplification at low temperature before the first denaturation step of the PCR. The hot start system includes the hot start DNA polymerase, or a composition for the inactivation of the DNA polymerase in the storage solution and its activation under heating and the protection for primer-dimer formation i.e by means of primer binding to protein as proposed for example in the HotStart-IT (USB Europe GmbH, Staufen, Germany).

The inventors have found that the present invention has the following features which perfectly respond to the technical requirements of such assay as stressed in the invention summary. The heat exchange is compatible with the rapid changes of temperatures required in the PCR cycles. This was unexpected for a large and flat chamber since any small delay or imprecision on the temperature or unhomogeneity along the chamber would impair the efficiency of a cycle and would lead to a much lower PCR yield over the successive cycles. Constraint on the flat surface having a large thickness is not obvious to fulfil and could not be obtained for example with a Peltier element commonly used in the PCR cycler The difficulty encountered by these constraints is that the surface has to be large enough in order to contain enough locations for the detection of multiple targets. Typical flat surfaces are bigger than 0.2 cm² or preferably bigger than 1 cm² or even more preferably bigger than 2 cm². The constraints on the heat exchanges on the PCR efficiency is very high since the time for each of the 3 temperature steps used in an amplification cycle has to be limited to between 0.5 to 2 min in order to make the assay competitive in term of time. The second constraint is the fact that the amplification is a repetitive assay and a small decrease in efficiency of one cycle would result in a large drop of the final yield at the end of the amplification cycles. This feature allows performing the PCR on a flat surface where the detection takes place, thus allowing PCR-array detection and even real-time PCR on micro-array. Another requirement of the present invention is the fact that the support stays perfectly flat throughout the process which makes possible the detection of the multiple locations. The flatness is necessary both for the illumination and for the emitted light. This invention allows detection to be performed in one step on the overall surface or in a scanning mode. The difficulty encounters by these constraints is that the surface has to be rather thick for supporting the high temperatures and the rapid changes of temperatures due to the PCR amplification and has to be large enough in order to contain enough locations for the detection of multiple targets.

The present invention gives an easy solution to an unexpected problem linked to the constraints of the present complex method which is to keep during the PCR a homogeneous reaction solution. The present process allows removing air bubbles from the solution so that the surface bearing the target has a homogeneous interface with the solution both during the reaction of the target with its capture probe and for its detection. This does not require any additional handling step thus making the detection to be possible during the PCR cycles. This feature is essential of the present method where multiple targets have to be detected and measured so that the measurement performed on one target present in one localized area of the surface can be compared to the measurement of another target bound to another localized area of the surface. Thus the invention provides an easy solution to the constraint of the formation of bubbles of air during the heating which would interfere with the detection of the targets bound to their capture molecules.

Another feature of the invention is the absence of constraint of contact between the device and the solid support so that the user is free to use any detection means for excitation and emission of labelled targets present on the surface of the support. Such detection when performed preferentially in fluorescence needs both the excitation and emission beams coming or detected outside the chamber to reach all the surface of the support where bound targets are present. In this invention, the heating process of the PCR does not interfere with the detection of the signal coming from the surface of the support. This feature makes possible detection of target polynucleotide molecules bound to corresponding capture molecules during the PCR cycles, i.e. in a real-time PCR.

In a preferred embodiment, the signal is the result of light emission (8) from the bound labelled target polynucleotide molecule in response to excitation light (7).

In another embodiment, the surface of the reaction chamber having fixed capture molecule is transparent to the excitation and/or emission light.

In an alternative embodiment, at least one side and/or the top ( cover) of the reaction chamber is in a material transparent to the excitation and/or emission light.

In a preferred embodiment, the measurement of the labelled target polynucleotide molecule is performed at least once during the PCR amplification.

Preferably, the measurement of the labelled target polynucleotide molecule is performed in at least 5, preferably at least 10 thermal cycles and even preferably at least 20 thermal cycles.

Preferably, the assay is a real-time PCR including PCR and multiple detections of amplicons produced at different thermal cycles.

The present invention provides a PCR efficiency per cycle of at least 60%, and even of at least 80% and even of at least 90% compared to the PCR obtained in a PCR tubes and performed in a standard PCR cycler (Peltier thermocycler). The amplification yield at the end of the PCR is preferably at least 20% and preferably 50% and even more preferably more than 80 % compared to a PCR performed in a tube in a standard PCR cycler.

Advantageously, the invention provides a PCR efficiency of at least 60%, and even of at least 80% and even of at least 90% in the linear amplification cycles of the PCR.

One of the main features of the present invention is the high efficiency of the PCR amplification. The efficiency of the PCR is depending on many parameters such as the specificity of the primers, the choice of the annealing and elongation temperatures, the stringency of the solution, the activity of the DNA polymerase, the concentrations of the primers, dNTP and the duration of each PCR temperature step.

The efficiency is maximal when 100% of the target molecules are copied in one cycle. Such efficiency can be reached in the first cycles of the PCR when the parameters of the reaction are optimum. In such a case it is easy to calculate the amplification factor since it will be 2ⁿ, n being the cycle number. This is valid only in the first part of the PCR cycles when the amplification is logarithmic. In the latest cycles, the efficiency always decreases.

Such maximal efficiency requires that all target molecules are amplified in each cycle. One condition is that the three steps: denaturation, annealing of the primers, elongation of the amplicons occur for all the targets present at this particular cycle. Any restriction with temperature or unhomogeneity of the temperature in the solution will lower such efficiency since it is a limitative effect. A small loss of 10% of efficiency in one cycle will lead to a final yield of 35 % after 10 cycles, of 12.3 % after 20 cycles, of 4.2% after 30 cycles and of 1.5% after 40 cycles. The results in figure 6 show a small decrease in the signal of the hybridized amplicons after 40 cycles when the PCR is performed on a flat surface 500 µm thick as compared to PCR tubes. The signals obtained with the flat surface chamber of 500 µm were between 5% and 100% with a mean of 64% compared to the PCR tubes depending on the specific sequence amplified. This experiment was performed with very low amount of targets ranging between 40 and 80 copies per PCR reaction. With such low copy number, the amplification is linear in the at least 30 first cycles. If the signals reflects the amount of amplicons, we can conclude that the present invention give an average PCR efficiency of more than 90% for the amplified targets.

In the method of the invention, the PCR is performed when the reaction chamber (2) is subjected to a rotating movement. Rotation is preferably performed at a speed ranging from 50 to 5000 rpm, preferably between 100 and 1000 rpm.

In a preferred embodiment, the PCR is performed with the rotating holder (1) being rotated at a speed of at least 100, preferably 400 and still preferably 1000 rpm. The accuracy on the rotation speed is ± 20 rpm. The braking time needed to stop the rotor is preferably lower than 3s.

The rotation is sufficient in order to develop a centrifugal force in the reaction chamber so that the air bubbles are removed from the inner chamber and/or do not interfere with the light excitation or/and emission necessary for the detection of the bound target. Preferably, the height of the liquid in the detection part of the reaction chamber is usually very small to avoid the use of large sample volume. The height of the liquid in the reaction chamber in the detection part of the chamber is preferably comprised y between 0.1 and 5 mm, and even more preferably between 0.2 and 2 mm. In this condition, the air bubbles formed during the PCR cycles have the tendency to stick to the surface thus preventing the hybridization of the amplicons on their capture molecules and interfering with the detection of the bound targets.

In a preferred embodiment, the surface bearing the bound capture molecules has a homogeneous interface after a PCR cycle.

In another embodiment, the interface between the surface containing bound target and the solution is homogeneous in at least 90% of the surface.

In a preferred embodiment, subjecting the reaction chamber to a rotating movement during the thermal cycles prevents a presence of bubbles on the localized areas.

While bubbles may be employed to mix the hybridization fluid during hybridization, e.g., as described in U.S. patent application Ser. No. 09/137,963, bubbles are undesirable in hybridizations where a thin film of hybridization fluid is used, for a number of reasons. Bubbles are local inhomogeneities in the hybridization fluid. And if they remain substantially immobile during hybridization, the bubbles can result in uneven heat transfer and localized hot spots on the surface containing bound biomolecules. Bubbles may disrupt the continuity interface between the hybridization fluid and the surface containing bound probes, thereby displacing fluid away from surface-bound probes and preventing target species from contact with the probes.

In a preferred embodiment, the measurement of the labelled target polynucleotide is performed with the reaction chamber being on said rotating holder.

In another embodiment, the measurement of the labelled target polynucleotide molecule is performed in presence of the amplification solution containing the labelled target polynucleotide molecules (being present in the same reaction chamber). The method avoids removing the solution from the surface of the support carrying a micro-array and avoids washing before the measurement. The washing includes liquid handling of the solution containing amplified target and possible contamination of further assays in the laboratories.

In another embodiment, the measurement of the labelled target polynucleotide molecule is performed when the chamber is subjected to a rotating movement. Rotation is then preferably performed at a speed ranging from 2 to 5000 rpm, preferably between 100 and 1000 rpm. Advantageously, the localized areas are scanned meanwhile the support is rotating upon its axis.

In an alternative embodiment, the measurement of the labelled target polynucleotide molecule is performed when the chamber is not subjected to a rotating movement. The rotor is stopped and the localized areas are detected, preferably all the localized area are detected in one shot, preferably with a CCD camera.

In one embodiment, centrifugation is also used to remove the solution from the reaction chamber to a second reaction chamber or reservoir, allowing the measurement of the bound labelled target polynucleotide molecules in absence of solution comprising the labelled target polynucleotide molecules. In a preferred embodiment, the solution containing the labelled target polynucleotide molecules is moved from the reaction chamber to a second reaction chamber by centrifugation. Preferably, reading of the bound labelled target polynucleotide molecules is performed in the reaction chamber in absence of solution comprising the labelled target polynucleotide molecules. The reaction chamber is preferably in fluidic communication with a second chamber forming a closed cartridge. The cartridge may be pivoted of 180° on the holding rotor, so that the g force pushes the solution from the reaction chamber to the second reaction chamber. Preferably, reading of the bound labelled target polynucleotide molecules is performed in the reaction chamber in absence of solution comprising the labelled target polynucleotide molecules.

Advantageously, the method of the invention does not require the use of different fluorescent dyes to quantify different polynucleotide molecules One fluorescent dye is sufficient for the quantification of multiple different polynucleotide molecules because of their specific binding by hybridization on capture molecules being specific of each target polynucleotide sequence and being localized in distinct areas of the micro-array. For example, a polynucleotide molecule is amplified together with another polynucleotide molecule using the same or different primers and both amplicons are labelled with the same fluorescent dye. The different amplicons are detected and/or quantified on separated capture molecules without the need of several fluorescent dyes as required in the real time solution PCR.

Another advantage of the method is its great specificity. A first specificity level is obtained through the annealing of the primers and a second level of specificity is obtained by the hybridization on the capture molecules. Such double specificity increases very much the specificity of the final detection which is often required for analysis in complex biological sample. Another advantage is that primer dimers or non specific amplified product formed during the PCR amplification will not generate signal on the micro-array since there is no complementary capture molecules for the primers nor for unspecific products.

The specificity can still be increased by the use of different capture molecules for the same target polynucleotide molecule. Two or more capture molecules can be designed to bind the same strand or one capture probe will bind the sense strand of the amplified product and another capture molecule the antisense strand.

In a preferred embodiment, the capture molecules are bound to a localized area of the flat surface in the form of a micro-array. Preferably, the micro-array comprises more than 5 different capture molecules (20), preferably more than 20 and even more than 50.

In a preferred embodiment, the localized area is comprised between about 10 µm² and about 1 mm² and preferably between about 1 µm² and about 100 µm².

In the invention, the capture molecules present on the micro-array are complementary to at least one part of the sequence of amplified target polynucleotide sequence present in solution. The capture molecules comprise a polynucleotide sequence which is able to specifically bind the amplified target polynucleotide sequence, said specific polynucleotide sequence is preferably separated from the surface of the solid support by a spacer arm of at least about 6.8 nm or of 20 nucleotides and preferably of 40 nucleotides preferably in a double stranded form which has no binding affinity for the amplified target molecule. In a preferred embodiment, the capture molecule is a single stranded polynucleotide containing a sequence able to specifically bind the labelled target polynucleotide molecule and a spacer of at least 6.8nm long being preferably a sequence of at least 20 nucleotides and preferably more than 40 nucleotides long.

In a preferred embodiment the probe sequence specific for the target binding is comprised between 15 and 100 nucleotides and more preferably between 15 and 35 nucleotides.

In the preferred embodiment, the polynucleotides being used as capture molecule are between 10 and 1000 nucleotide long and preferably between 100 and 400 nucleotides long. For specific binding of homologous sequences possibly present in the same sample, the polynucleotide capture molecules contain a spacer according to the patent WO017737 Specific binding of homologous sequences or SNP possibly present in the same sample, are obtained using capture molecules having a specific part being between 15 and 30 nucleotides.

In the preferred embodiment, the polynucleotides being used as capture molecules are present on the micro-array localized area at a density superior to 10 fmoles, and preferably 100 fmoles per cm² surface of the solid support.

The micro-array according to this invention contains between 4 and 100,000 spots per cm² and preferably between 20 and 1000 spots per cm², each spot being the localized area for one capture molecule. Miniaturization allows performing one assay onto a surface (usually circular spots of about 0.1 to about 1 mm diameter). A low density array, containing 20 to 400 spots is easily obtained with pins of 0.25 mm at low cost. Higher density of spots going to 1,600 spots per cm2 can be obtained by reducing the size of the spots for example to 0.15 mm. Method for obtaining capture molecules of higher density have been described earlier as in US 5,445,934. Miniaturization of the spot size allows obtaining a high number of data which can be obtained and analyzed simultaneously, the possibility to perform replicates and the small amount of biological sample necessary for the assay. Miniaturization for detection on micro-arrays is preferably associated with microfluidic substrate for separation, extraction of polynucleotide molecules from the cell extract.

The invention further relates to a method for multiplex PCR amplification of two or more target nucleic acid sequences having a different sequence composition.

In a preferred embodiment, the reagents for polynucleotide molecule amplification comprises at least 5 primer pairs, preferably at least 10 primer pairs, more preferably at least 20 primer pairs et even at least 40 primer pairs.

In an embodiment, the micro-array is in contact with reagents for carrying out the amplification of one or more polynucleotide sequences. In another embodiment, between 1 and 4 polynucleotide molecules and preferably between 1 and 20 polynucleotide molecules present in a solution are amplified and detected and/or quantified in the same assay. Preferably, between 20 and 1000 polynucleotide molecules present in a solution are amplified and detected and/or quantified in the same assay.

Advantageously, the polynucleotide molecules to be amplified are homologous polynucleotide sequences which are quantified on micro-array during the PCR using consensus primers as described in WO017737 The same primers are used to amplify all the homologous sequences possibly present in a sample. The amplicons which are labelled with the same fluorescent dye are discriminated on different capture molecules, each one targeting a different homologous sequence. So with only one primer pair and one fluorescent dye, the assay is rendered multiplex by the use of multiple capture probes present on the micro-array.

In one embodiment the number of sequences amplified by the same primer pair is higher than 2 and even higher than 5 and even higher than 20. The amplified targets are then detected on the array.

In another embodiment, standards polynucleotide sequences are incorporated into the tested solution and the standards are amplified with the same primers as the target polynucleotide sequences.

In the main embodiment, target and/or capture molecules are polynucleotides. The capture molecules are attached preferably by covalent link on the support or substrate present on the support. In another embodiment, the capture molecules are adsorbed on the support as long as they are not significantly released in solution during the PCR cycles.

Deposition of the capture probe is preferentially done with physical means such as pin or "pin and ring" touching the surface, or by release of a micro-droplet of solution by methods such as piezo or nanodispenser. Alternatively, *in situ* synthesis of capture molecules provides a high spatial resolution of the synthesis of oligonucleotides or polynucleotides in known locations such as provided by 5,744,305 and 6,346,413.

In another embodiment the polynucleotide molecules are DNA present in a biological sample.

The DNA is extracted from the sample and amplified by PCR and the amplicons are detected online by their fixation on their specific capture molecules. In one particular embodiment, the polynucleotide molecules are homologous polynucleotide sequences which are detected and/or quantified online on micro-array after amplification of genomic DNA by consensus primers as described in WO0177372.

According to the invention, the solid support used for the construction of micro-array is preferably selected from the group consisting of glass, metallic supports, polymeric supports (preferably thermo-resistant having low self-fluorescence) or a mixture thereof in format such as slides, disk, multi-well plates, strips, gel layers, and microbeads. The solid support is preferably a support used in the microchips (or micro-arrays) technology (preferably activated glass bearing aldehyde or epoxide or acrylate groups), said support comprising also specific coatings, markers or devices (bar codes, electronic devices, etc.) for improving the assay.

If glass presents many advantages (like being inert and having a low self-fluorescence), other supports like polymers, with various chemically well-defined groups at their surface, allowing the binding of the polynucleotide sequences are useful. In another preferred embodiment, the support bearing the capture molecules has a 3 dimensional porous structure. Conventional glass slides have less than 60% silicon dioxide on their surface. This inherently limits the amount of chemical bonding available on the surface. Porous material exhibits increased loading capacity of capture molecules. Typical porous supports are gel pads, fused-fiber matrix, fibrous polymer matrix. The array can be constructed entirely of the porous material, or can comprise a layer of porous material mounted on top of a flat surface such as glass, plastic, or metal.

In another embodiment capture molecules are present on different supports being preferentially beads with chemical or physical characteristics for their identification with a specific capture molecule.

In still another embodiment, the support bears several micro-arrays separated by physical or chemical boundaries. Examples for physical barriers are wells, e.g. the support being a 96, 384, 1536 multi-well plate, thus creating separated localized areas onto which capture molecules may be spotted individually. 384-well and 1536-well plates are available from BD Falcon for cell based assays (Merck Eurolab sa, Leuven, Belgium) or from Nunc A/S (Roskilde, Denmark). 6144 format microtiter plates are available from Parallel Synthesis Technologies Inc. (PSTI, Menlo Park, CA, USA). The multiwells are present as one plate or in strips. Other physical barriers are tubes such as 96, 384, 1536 or even 6144 tubes deposit at the surface of the support. Tubes are similar to the well formats but do not have a plain bottom so that when deposit on the surface of the support, they create localized areas isolated from each other. An example for a chemical barrier is e.g described in DE 0019949735A1, where defined areas within a hydrophobic surface are provided with hydrophilic anchors allowing the precise location and confinement of capture molecules on a solid support.

In a preferred embodiment, the rotating holder (1) has a disk shape. In another embodiment, the rotating holder (1) is the rotor.

The rotating holder (1) is preferably adapted to fit the support format being preferably a flat plastic reaction chamber having an inlet and a flat surface with the immobilized capture molecules. In another embodiment, the holder is adapted for a 96 wells mitrotiter plate or a single well or tube.

The rotating holder preferably contains more than 1 reaction chamber and preferably 4 or even more than 8. The number of reaction chamber is adapted to avoid any resonant frequency during the accelerating phase from 0 rpm to 1,000 rpm and during the running phases at constant speed.
Preferably the speed of the rotation is higher than 100 rpm and preferably comprised between 400 and 1000 rpm with an equivalent rotation diameter of 110 mm. The holder preferably has preferably an aperture on the external part of the holder chamber. Preferably the holder is submitted to vibration while rotating. Preferably the reaction chamber has two positions on the holder, each position being oriented at 180 % from each other. The purpose is to place either the reaction chamber or the second reaction chamber on the outside of the rotor in order to centrifuge contained liquid in one direction of the other. Preferably the liquid of the chamber is transferred from one chamber to the other according to the position of the chamber relative to the turning axis of the rotor.

The rotating holder is capable to be stopped accurately at a position that allows laser illumination and camera reading of the reaction with an accuracy of +/- 0.5° (+/- 0.5 mm for a diameter of 110 mm). The accuracy of positioning of the reaction chamber is preferably better than 2 mm and even better than 0.5 mm. Preferably, the rotor is not rotating during the detection process of the bound targets.

The rotating holder has preferably a diameter between 110 and 200 mm.

The reaction chamber is placed and tightly locked in the rotating holder, the lodge or cavity where it is inserted is preferably made of a non-reflective and light absorbent material to avoid interference with the illumination or detection of the array.

Advantageously, the reaction chamber (2) is preferably part of a disk support to allow an easy centrifugation of the reaction chamber during the PCR. A general scheme of the integrated apparatus comprising the rotating holder (1) having a disk shape, a reaction chamber (2) comprising capture molecules (20) immobilized in localized areas (21) of a flat surface of the support, temperature regulating device (5) based on hot pulsed air (6) and a detector (9) is presented in figure 1 to 4.

A typical assay is performed preferably in the following way. In a first step, a solution containing the polynucleotide molecules and reagents for amplification and labelling are introduced into the reaction chamber (2) being fixed on the rotating holder (1) having a disk shape. In step 2, the reaction chamber is sealed. In step 3, the rotating holder (1) is centrifuged and the PCR amplification is performed in the reaction chamber (2) while rotating. The disc rotates on its axe preferably by the means of a step motor. The change in temperature in the reaction chamber is obtained by changing the temperature of the air around the reaction chamber using hot pulsed air (6). In a preferred embodiment, changing the temperature of the air around the reaction chamber is obtained by (hot) pulsed air (6). Rotating the support during the PCR allows uniform thermal regulation of the reaction chamber (2). In step 4, the rotor is stopped and the labelled target molecules bound to the capture molecules are measured through light excitation (7) and detection of the emitted light (8) by the detector (9). In another embodiment, the detection is performed during the rotation of the rotor preferably in a scanning mode.

In a preferred embodiment, the rotating holder (1) bears several micro-arrays separated by physical boundaries, preferably the rotating holder (1) has a multi-well plate or strip format. In another embodiment, the multi-well plate is submitted to a temperature gradient during the measurement of light emission (8).

In a preferred embodiment, the reaction chamber contains 2 or preferably 3 compartments being in fluid connection to each other and comprising a flat surface carrying the micro-array. The support is preferably made of a plastic slide covered with a flow through observation channel where the micro-array is build up. The observation channel is terminated by one or two reservoirs preferably located at both sides. One reservoir is preferably used to introduce the solution and the other one to remove it. The reservoirs are sealed by specific lids to avoid the evaporation of the solution during thermal cycles. In a particular embodiment, the solution is moved over the micro-array in order to increase the speed of the binding reaction of the labelled target polynucleotide molecule on its capture molecule. This is obtained by rotating, translating or moving up and down the reaction chamber during at least the annealing step of the thermal cycle. In still another embodiment, the height of the liquid on the surface having fixed the micro-array is lower than 1 mm and preferably lower than 0.1 mm and even more preferably lower than 0.02 mm.

Detectable labels suitable for use in the present invention include any composition detectable by eletromagnetic light emission. In an embodiment, the target molecules are labelled with a fluorescent dye. The fluorescent label can be incorporated into the target by enzymatic or chemical reaction. Typical enzyme reaction includes the incorporation of polynucleotide analogues into the target. Alternatively, primers labelled at their 5' end with a fluorescent dye can be incorporated into the target. Fluorochromes can also be incorporated into the targets by chemical reaction such as the reaction of fluorescent dye bearing a N-hydroxysuccinimide (NHS) group with amines groups of the targets. Useful fluorescent dyes in the present invention include cyanine dyes (Cy3, Cy5, Cy7), fluorescein, texas red, rhodamine, green fluorescent protein. Preferably, the excitation wavelength for cyanin 3 is comprised between 540 and 558 nm with a peak at 550 nm and the emission wavelength is comprised between 562 and 580 nm with a peak at 570 nm.

Preferably, the excitation wavelength for cyanin 5 is comprised between 639 and 659 nm with a peak at 649 nm and the emission wavelength is comprised between 665 and 685 nm with a peak at 670 nm. Fluorescent dyes equivalent to cyanin 5 are the Oyster 645 and Quasar 670. The excitation wavelength for Oyster 645 is comprised between 635 and 660 nm with a peak at 645 nm and the emission wavelength is comprised between 650 and 680 nm with a peak at 666 nm. The excitation wavelength for Quasar 670 is comprised between 635 and 665 nm with a peak at 644 nm and the emission wavelength is comprised between 660 and 685 nm with a peak at 670 nm.

Preferably, the excitation wavelength for cyanin 7 is comprised between 733 and 753 nm with a peak at 743 nm and the emission wavelength is comprised between 757 and 777 nm with a peak at 767 nm.

Patents teaching the use of such labels include U.S. Patent Nos. 3,817,837; 3,850,752; 3,939,350; 3,996,345; 4,277,437; 4,275,149; and 4,366,241.

Some fluorescent labels may be of particular interest, such as nanocrystals particles having fluorescent properties. The most common one are the Quantum dots (Han et al., Nature Biotechnology 19, 631-635, 2001). They are fluorescent and do not bleach with time or with illumination. Their stability makes them particularly suitable for the use in continuous reading as proposed in this invention. Also, they contain metals which confer to these particles specific properties so that other methods than the fluorescence can be used to follow their attachment on the capture probes. Thermal heating of these particles is one of the parameters that may be followed with time. The fact that the metal absorbed the energy of a light beams preferably a laser and induce a heating of the particle has been used as a basis for the detection of low density gold particle on a support and even single particles are detected (Boyer et al Science, 297,1160-2002). The method is called Photothermal Interference contrast.

Another technology for the direct measurement of nanoparticles is the Rayleigh Scattering. This method is based on the use of a light beam adapted in order to obtain an oscillation of the electrons in the metal particle so that an electromagnetic radiation is obtain from the particle which can be detected. (Stimpson et al., Proc. Natl. Acad. Sci. USA 100 (2003), 11350-11353) (real-time detection of DNA hybridization and melting on oligonucleotide arrays by using optical wave guides) The method is lacking sensitivity for the applications on biological samples.

Alternatively, Raman scattering and the surface plasmon resonance may be applied in the present invention, which technique has been extensively used for the detection of antibody/antigen binding but are also well suited for the multiparametric measurement of the arrays and for the required sensitivity on biological samples. (Thiel et al., Analytical Chemistry, 69 (1997), 4948-4956).

In another embodiment, quartz crystal microbalances may be applied, which are now sensitive enough that they can measure changes of mass lower than nanogram (cf. Caruso et al., Analytical Chemistry 69 (1997), 2043-2049). This is one proposal for micro-array detection in real-time.
Cantilevers are another option for the detection of DNA on micro-arrays. (McKendry et al. Proc. Natl. Acad. Sci. USA, 99 (2002), 9783-9788).

Also, another technology is the electrical detection of the nanoparticles which takes into account their metal properties. The electrochemical detection was first applied but with low sensitivity.
The more advanced and sensitive method is the detection by differential pulse voltametry (Ozsoz et al., Analytical Chemistry 75 (2003), 2181-2197).

The resistivity and the capacitance properties of the metal are also one of the best properties to be detected on electronic chips. The presence of a metal between two electrodes will induce a change of resistivity and of capacitance. The detection of the DNA or proteins is then observed when the capture molecules are present on one of the electrode (Moreno-Hagelsieb et al Sensors and Actuators B-Chemical, 98, 269-274, 2004). The capacitance assay of the gold labelled DNA has been described by Guiducci et al. ESSDERC 2002. Since electronic chips can be made of several plots, different targets may be detected on different plots and the change in the resistivity or in the capacitance may be recorded. If the methods have not yet been able to produce reliable and sensitive detections as required by the biological samples, it is, however, predicted that some of them will succeed to fulfil the requirements for the real-time detection.

Another promising technology for measuring the binding of the target molecules on capture molecule of the micro-array is the chemical cartography based on optical process of non-linear generation frequency spectroscopy (GFS) (L. Dreesen et al. Chem Phys Chem, 5 , 1719-1725, 2004). This technology allows the imaging in real time of the vibrational properties of surfaces and interfaces with a submicron spacial resolution. The measurement is obtained by mixing at the surface of a substrate two laser beams, one having a fixed frequency in the visible (green) and the other having a variable frequency in infrared. The vibrational signature at the interface is obtained by measuring the light emitted by the sample in function of the frequency of the infrared laser beam. This method allows avoiding labelling of the target in order to be detected.

The original polynucleotide molecule is not necessary labelled before the amplification but lead to amplified labelled target molecules during the amplification step.

The amplified polynucleotide molecules are able to hybridize on the capture molecules after a denaturation step. As the amplified polynucleotide molecules are double stranded, in theory they must reassociate in solution much faster than to hybridise on capture molecules fixed on a solid support where diffusion is low and the specific binding sequence is short, thus reducing even more the rate of reaction. Therefore, it was unexpected to observe a significant signal increase on the capture molecules over multiple thermal cycles after a short period of incubation time.

In a particular embodiment the measurement is performed on bound target labelled molecules while they reassociate in a double stranded form in the solution during annealing and/or elongation of the thermal cycle.

In a preferred embodiment, the reagents for polynucleotide molecule amplification comprise a primer pair, dNTPs, a thermostable DNA polymerase, a hot start PCR system and buffer. The hot start PCR system maximizes PCR efficiency by minimizing unwanted non-specific hybridization and extension of primers or formation of primer-dimers. In a preferred embodiment, the hot start PCR system is a hot start DNA polymerase.

The 3 different temperatures steps of the PCR allow denaturation of the double strand polynucleotides, annealing of the primers and elongation of the primers by a DNA polymerase.
In a preferred embodiment, the annealing time of the PCR is at least 60 sec and preferably 90 sec.

In another embodiment, the denaturation and/or elongation time of the PCR is at least 30 sec, preferably 45 sec and even preferably 60 sec.

In another embodiment, a thermal cycle comprises a temperature step of hybridization allowing a specific hybridization between said target polynucleotide molecule and its corresponding capture molecule (20).

Preferably, each of the temperature steps of denaturation, annealing, elongation and hybridization is performed in 1 min or less, preferably in 30 sec or less.

In a particular embodiment, the assay is performed in a continuous or semi-continuous way over the annealing and/or elongation and/or denaturation step.

In a preferred embodiment, the reagents for polynucleotide molecule amplification comprise a primer and/or dNTP labelled with a fluorescent dye, preferably selected from the group consisting of: Cyanin 5, Quasar 670 and Oyster 645.

In a preferred embodiment, the flat surface is at least 0.04 cm² or preferably at least 1 cm² or even more than 2 cm². In another embodiment, the flat surface is at least 0.25 mm thick and preferably 0.5 mm thick.

In another embodiment, at least one part of the reaction chamber is in a (heat) conductive material. There is now the possibility to incorporate conductive substances even in polymer material in order to make them preferably heat conductive. Since these conductive substances are usually opaque to light they can not be used for all the chamber walls but at least for some of them.

In another embodiment, the height of solution in the reaction chamber (thickness of solution above the capture molecules) is comprised between 50 µm and 500 µm. The volume of solution in the reaction chamber is preferably comprised between 10 µl and 200 µl.

In another preferred embodiment, the flatness of the surface is changed by less than 0.05 mm after at least 20 amplification cycles.

In an alternative embodiment, the solution composition is adapted for performing the annealing of the primers on the polynucleotide molecule and the hybridization of the labelled target molecule on the capture molecule during the same temperature step.

In still another embodiment, the steps of denaturation, annealing, elongation are performed in 1 min or less. In still another embodiment, the hybridization and the annealing are performed in the same step.

In a preferred embodiment, the Tm of the primers for a target are within a range of the temperature of annealing -2 to +8°C and preferably plus 0 to +4°C. The Tm is preferably calculated taking into account the thermodynamic values of Santa-Lucia, 1998 (Proc. Natl. Acad. Sci; USA, 95, 1460-1465) corrected for the salt concentration by Owczarzy et al., 2004, (Biochemistry 43, 3537-3554).

In another preferred embodiment, the Tm of the capture molecule for a target is within a range of temperature of the hybridization plus 4 to 16°C and preferably plus 8 to 12°C. In still another preferred embodiment, the Tm of the two capture molecules differing from one base and use for the discrimination of a SNP in a target sequence have a Tm within a range of temperature of hybridization plus 4 to 8°C.

A particular aspect of the invention is a method for PCR amplification and detection of a polynucleotide molecule being present in a solution contained in a chamber having a surface bearing the capture molecules for the detection of the amplified sequences comprising the steps of:
- providing a rotating holder (1) and a reaction chamber (2) having fixed upon one of its surface at least a capture molecule (20) being immobilized in a localized areas (21) of a flat surface of said reaction chamber,
- introducing a solution containing said polynucleotide molecule into said reaction chamber (2) and reagents for polynucleotide molecule amplification and labelling,
- submitting the solution to at least 2 thermal cycles comprising a denaturation, annealing and elongation steps in order to obtain labelled target polynucleotide molecule by PCR amplification,
- performing at least a measurement of the labelled target polynucleotide molecule in the following way,
   - incubating said labelled target polynucleotide molecule present in said solution under conditions allowing a specific hybridization between said target polynucleotide molecule and its corresponding capture molecule (20),
   - measuring a signal originating from the surface having the bound labelled target polynucleotide molecule in response to illumination of said flat surface, wherein the signal is measured outside the chamber, and wherein the surface of signal emission comprises at least one localized area having a surface of between about 0.1 µm² and about 75 mm²,
      wherein the said surface has a homogeneous interface during the measurement of the signal,
   - wherein the hybridization and the annealing steps occur at the same temperature and wherein the Tm of the primers for a target are within a range of the temperature of annealing plus 0 to 4°C and the Tm of the probe for said target is within a range of temperature of the hybridization plus 6 to 12°C and
- Processing the data obtained in order to detect and/or quantify the amount of polynucleotide molecule present in the solution before the amplification.

In another particular embodiment, the hybridization, annealing and elongation are performed in the same step. In still another embodiment, the steps of annealing and hybridization are performed in 2 min or less. In a preferred embodiment, the Tm of the capture molecule for a target is at least 4°C and preferably 6°C higher than the Tm of the two primers specific of said target. In a particular embodiment, all the capture molecules and primers of the targets to be detected have Tm within the range given here above.

In a specific application, the annealing and hybridization temperature are fixed at 60°C with primers having a Tm of about 64°C and the probes at around 70°C for a target.

In a preferred embodiment, the thermostable DNA polymerase used for PCR on micro-array is the hot Master (Eppendorf, Hamburg, Germany) which works at 62°C. In a preferred embodiment the steps of annealing, elongation and hybridization on the array are performed at the same temperature which is comprised between 60 and 68°C. Advantageously, the method of the invention is compatible with most of the thermostable DNA polymerase available on the market. It does not necessary require a 5' to 3' nuclease activity as described in the U.S. Pat. No. 5,952,202.

Preferably, the PCR is performed using a DNA polymerase having a strong DNA binding domain preferably the Helix-hairpin-Helix (HhH) of the topoisomerase preferably the TopoTaq DNA polymerase. In another preferred embodiment, the enzyme contains more than one DNA binding domain, preferably having at least three DNA binding domains and more preferably 5. Also a preferred DNA polymerase is a chimeric polymerase which is active at higher salt concentrations. The optimum elongation temperature for this enzyme is 72°C. The present DNA polymerase allows the amplification of sequences longer than 200 bp and longer than 400 bp using a DNA polymerase having a strong DNA binding domain. Also the use of Betaine is advantageously long amplified sequences (amplicons) and/or when dealing with GC rich sequences.

In a preferred embodiment, the reagents for polynucleotide molecule amplification comprise a composition that allows a PCR amplification and a detection of target nucleic acids sequences to be performed in a same solution composition thus opening the possibility to perform a (PCR-array) amplification and detection in a same (single) medium and possibly in a same (single) reaction chamber of a device according the patent application EP07150423.7.

Preferably, the amplification composition is buffered to a pH comprised between 7 and 9 and comprises: at least 2 primer pairs, wherein each primer of the primer pair is present in the composition at a concentration lower than 100 nM, a thermostable DNA polymerase, a hot start PCR amplification system, a plurality of dNTPs, a salt composition having at least 100 and preferably 150 mM and even 200 mM of cation. Preferably the PCR solution contains a salt having a cation and an anion, wherein the said anion has two carboxylic groups and one amine group, wherein the salt concentration in the composition is comprised between 10 mM and 400 mM and from 1% to 20% by weight of an exclusion agent. The salt is preferably glutamate or aspartate.

In an embodiment, the solution contains 5' end labelled oligonucleotides or primers which serve as anchors for the polymerase to copy the target sequences to be detected on the micro-array.

In a preferred embodiment, the primers hybridize with the polynucleotide molecule in solution while the amplified target molecule obtained in a previous thermal cycle hybridize in the same time and in the same conditions, on a capture molecule being immobilized in a specifically localized area of a support. During the temperature step of elongation of a thermal cycle, the primers hybridized to the polynucleotide molecule are elongated in solution. The capture molecules (20) bound to the labelled target polynucleotide molecules are possibly elongated but not labelled. Alternate steps of annealing, elongation and denaturation during one cycle of reaction result in the accumulation of labelled products which hybridize on their capture molecule present on the micro-array but deshybrizes from their specific capture molecules after each denaturation cycle.

In a preferred embodiment, the labelled target polynucleotide molecules are specifically hybridized on their corresponding capture molecules (20) preferably during the temperature step of annealing and/or elongation.

In another embodiment, the labelled target polynucleotide molecules specifically bound on their corresponding capture molecules (20) are detected in a step different from the steps of annealing and/or elongation.

In another embodiment, the solution contains labelled dNTP which are incorporated by the polymerase into the target sequences to be detected on the micro-array.

In a particular embodiment, the targets are labelled by using labelled dNTP and the immobilized capture molecules (20) having bound to the target polynucleotide molecules are elongated and labelled during the PCR. Alternate steps of annealing, elongation and denaturation during reaction cycles result in the accumulation of labelled product being partly integrated into its specific capture molecule and which can be detected during one of the PCR steps or in a separate step.

In a particular embodiment, wherein the capture molecules (20) bound to the labelled target polynucleotide molecules are elongated during the temperature step of elongation.

In an embodiment, some capture molecules are elongated by the polymerase and some are in the same time hybridized with the amplified products which accumulate in solution during the thermal cycle. In one embodiment, the capture molecules elongated are detected during the temperature step of denaturation. In another embodiment, the capture molecules elongated and the labelled polynucleotide molecules bound on their capture molecule are both detected during the temperature step of annealing and/or elongation.

In a preferred embodiment, the hybridization is favoured over the elongation by using capture probes which are not capable of being elongated. In this case, capture molecules preferably include a base terminator or long stretch of identical bases at their 3' end such as polyA.
Alternatively, the capture molecules are immobilized on the support by their 3' end, the free 5' end being not able to be elongated by the polymerase.

In another embodiment, the elongation is favoured over the hybridization by performing PCR in the presence of one primer in excess and a reduced amount of the other primer.

In another embodiment, at the end of the thermal cycles, an annealing step of at least 10 min, and preferably at least 30 min and even more preferrably at least 60 min is performed in order to increase the signal of hybridization for polynucleotide molecules present at a very low concentration before the amplification.

In a preferred embodiment, a thermal cycle is performed within 10 min and better within 3 min and even better within 2 min. In an alternative embodiment, 30 thermal cycles are performed within 5 h and better within 3 h and even better within 1.5 h.

Advantageously, the length of the amplified target polynucleotide molecules are selected as being of a limited length preferably between 80 and 800 bases, preferably between 80 and 400 bases and more preferably between 100 and 200 bases. This preferred requirement depends on the possibility to find primers to amplify the required sequences possibly present in the sample. Too long target may reallocate faster and adopt secondary structures which can inhibit the fixation on the capture polynucleotide sequences.

In a preferred embodiment, an excitation light (7) from a light source is directed on the surface of the support. Thus, the detected light is the light emitted by the bound target molecule under excitation from a light source.

The method is particularly well fitted to control the light excitation with the light directed on the surface of the chamber and the homogeneity of the excitation at each localized area can be determined and corrected if necessary.

In a preferred embodiment, the light beam is a laser beam which is focused on the surface of the micro-array in order to excite directly the fluorescent target molecules. The laser beam is preferably focused perpendicular to the surface of the array either through the solution or through the support. The emitted light is detected in the opposite direction of the excitation laser beam. The emitted light is preferably detected as a confocal light and measure after amplification by a photomultiplier.

In a preferred embodiment, the excitation light is the result of a light beam focused on the surface of the chamber having bound capture molecules as provided in figure 2.

In a preferred embodiment, the surface of the micro-array is scanned by the laser beam in order to obtain a maximum light excitation of the bound targets. In another preferred embodiment, the emission light is measured simultaneously from more than one localized area (preferably all the localized areas) using a CCD camera.

In a preferred embodiment, as provided in figure 4, the detected signal is the measured through the support bearing the bound capture molecules at an observation angle θobin relative to the normal to the said solid support surface in the support, such that 90° > θobin > critical angle θ_{c} [sin⁻¹ (n2/nl)], whereby the optically transparent solid support having a refractive index n1 and being in contact with a medium having refractive index n2, whereby n1 > n2. Preferably the observation angle is within the forbidden angle and being in the range of the critical angle θ_{c} plus 10°, preferably plus 5° and more preferably plus 3°.

Signal from bound target molecules is measured with a detector located at an angle 0° < θob out < θc out measured relative to the normal of the side of the transparent solid support bearing the capture molecules in the reaction chamber (2).
The angle θc out is linked to the angle θc by the following relations:
n1 sin (α) = n3 sin (θc out), where α is the angle formed by the normal (15) and the side of the transparent support (16) and n3 is the refractive index of the medium where the CCD camera is placed, typically air (n3 = 1)
Having in this example: α = (90° - θc ), the relation between θc and θc out becomes:
n1 sin (90 - θc) = n1 cos (θc) = n3 sin (θc out)
Thus: θc out = Arcsin (n1 / n3 cos (θc))
If n1 = 1.5, n2 = 1.33 and n3 = 1, then θc = 62.4° and θc out = 44.7°
In this case, the observation angle of the CCD camera outside the solid support must follow the following relation: 0° < θob out < 44.7°.

In another preferred embodiment, the light emission is due to evanescence excitation and/or to evanescence emission as provided in figure 3. In still another embodiment, the excitation light and/or the detected emitted light is totally internally reflected inside the transparent support. In a preferred embodiment, the light emission (8) is measured at a defined timing from the beginning of a temperature step.

In another preferred embodiment, the light emission (8) is measured within 5 min and preferably within 2 min and even more preferably within 1 min after the beginning of the annealing temperature step. In an alternative embodiment, the light emission (8) is measured at the end of at least one of the 3 temperature steps used for the PCR amplification.

In still another embodiment, the light emission (8) is measured at the end of the PCR amplification.

The micro-array is preferably scanned and each localized area is subsequently measured. Preferably the scanning of the array is performed within 1 min and preferably within 30 sec and even more preferably within 10 sec. Preferably, the scan of each localized areas is measured at the same precise moment of a temperature step when reading is repeated over multiple thermal cycles. Scanning is preferably performed by moving the light beam reaching the surface of the assay. However moving the array relative to the light beam is also another embodiment. Finally, moving boththe light beam and the array is one embodiment for the scanning. Example for scanning in the present invention is the scan of the array while rotating on the holder.

The fact that each localized area is subsequently measured can be advantageously used to monitor a kinetic of hybridization of a labelled target polynucleotide molecule on the same capture probe which has been immobilized at different localized areas of the support and which is scanned in a time dependant manner. Since the temperature is maintained constant during the measurement, the target polynucleotide molecule continues to hybridize on their capture probe during the scanning.

In a particular embodiment, the data on the quantification of the amplified target molecules performed at different PCR cycles are processed in order to quantify the amount of polynucleotide molecule present in the original solution before the amplification. The amplification cycles lead to the doubling of the target sequence in each cycle when the efficiency of the amplification is maximal. Quantification of the original polynucleotide concentration is calculated from the extrapolation of the first cycle which gives a detectable value or a value crossing a fixed threshold. The concentration is then calculated from a reference curve or from the data obtained on a standard molecule.

In a preferred embodiment, the signal associated with a capture molecule on the micro-array is quantified. The preferred method is the scanning of the array(s) with a scanner being preferentially a laser confocal scanner for the detection of fluorescent labelled targets. The resolution of the image is comprised between 1 and 500 µm and preferably between 5 and 50 µm.

In a preferred embodiment, the data are processed in order to obtain a signal value for each of the localized area. In another embodiment, the data are processed in order to obtain a signal value for each of the localized area and for the local background. The data are further processed by subtracting the background from the signal value for each of the localized area.

In a preferred embodiment, the quantification of the amount of polynucleotide molecule is performed by comparing the signal value of the localized area with a fixed value.

In an alternative embodiment, the quantification of the amount of polynucleotide molecule is performed by comparing the number of thermal cycles necessary to reach a fixed value (cycle threshold or CT) with the CT of a reference polynucleotide molecule. The reference polynucleotide molecule is preferably amplified in the same solution and detected on the same micro-array as the target polynucleotide molecule. Preferably, the polynucleotide molecule is labelled with a first fluorescent dye and the reference polynucleotide molecule is labelled with a second fluorescent dye different from the first fluorescent dye.

In another embodiment, the quantification of the amount of polynucleotide molecule is performed by comparing the number of thermal cycles necessary to reach a fixed value (CT) with a standard curve wherein the CT are plotted against standard concentrations. Preferably, two fluorescent dyes are used in the same solution.

The apparatus for monitoring on a micro-array a PCR amplification of a polynucleotide molecule being present in a solution comprises: a rotating holder (1) and a reaction chamber (2) having fixed upon its surface a micro-array, comprising at least one capture molecule (20) being immobilized in specifically localized areas (21) of a flat surface of said chamber; a thermal cycler for carrying out an automated PCR process; a rotor for rotating said holder (1) and said reaction chamber (2), an illumination light source and a detector (9) for measuring a signal from the bound labelled target polynucleotide molecule, with said solution being present in the chamber and containing the labelled target polynucleotide molecule wherein the surface of emission for a localized area is comprised between about 0.1 µm² and about 75 mm². Advantageously, the different parts are integrated into the same apparatus in order to read the signal of the bound labelled target polynucleotide molecule during the PCR amplification as provided in figure 8.

Preferably, the data of signal measurement are stored and treated for calculation of the amount or concentration of the different target molecules in solution and in the original biological sample. Data storage and data treatment are preferably performed using a programmable computer which is integrated in the apparatus of the invention.

The apparatus is controlled by a computer for the tasks of heating, rotation of the holder, possible pivot of the reaction chamber, signal measurement.
For example, commands to introduce have the following parametric input:
- temperature [°C],
- rotation speed of the rotation holder [rpm],
- possible pivot of the reaction chamber on the holder ([1] for reaction chamber - [0] for second reaction chamber),
- step time [sec] or reading time [sec],
- reading ([1] for reading - [0] for non reading),

For each step of the process (time starts running when the target temperature and rotation speed are reached).

If multiple reaction chambers are present on the rotating holder, all the reaction chambers are successively presented to the reading unit during the reading process.

Pictures taken during the reading process are preferably saved with the plastic device number and the time of reading (i.e. 03-080208-10:53:12 stands for plastic device n°3, picture taken the 8^{th} of February 2008 at 10h, 53 min and 12 sec). To save time, preferably only one picture will be taken, for each plastic device, during the reading step.

In a preferred embodiment, the apparatus further comprises:
- a storage system for storing the data of the different measurements for at least 5 localized areas of the support at a defined timing of a thermal cycle,
- a controller repeating the steps of illumination, detection and storage at least one time in at least one thermal cycle for each localized area of the micro-array,
- a program for processing the data obtained in at least one thermal cycle in order to detect and/or quantify the amount of polynucleotide molecule present in the sample before the amplification.

In a preferred embodiment, the rotating holder (1) has a disk shape. In another embodiment, the rotating holder (1) is the rotor. In still another embodiment, the rotating holder (1) comprises a plurality of reaction chambers (2).

In still another embodiment, the reaction chambers are able to rotate of 180° compared to the axis of the rotating holder.

In a preferred embodiment, the flat surface of the reaction chamber is thermostable and the surface is maintained flat at a temperature higher than 85°C and even higher than 94°C. The flat surface of the reaction chamber also presents a low self-fluorescence in order to be compatible fluorescence measurement.

In another embodiment, the micro-array comprises more than 5 different capture molecules (20), preferably more than 20 and even more than 50.

In another embodiment, the localized area is comprised between about 10 µm² and about 1 mm² and preferably between about 1 µm² and about 100 µm².

The apparatus may further comprise other means than rotation for mixing or agitating the solution inside the reaction chamber and increase the hybridization rate. Preferably, mixing is performed by moving the liquid in the reaction chamber by physical means such as pump, opening and closing valves, electrostatic waves, piezoelectric vibrations or mechanical vibrations. The frequency of vibrations is preferably the same as the rotation speed of the rotor. The amplitude of vibration is comprised between 0 and 2 mm with a maximum of 1.5 mm at 1000 rpm. Preferably, an unbalanced mass fixed on the rotor is used to create vibrations. Alternatively, an independent vibration device can be placed on the rotor motor for accurate control of vibration frequencies at all rotation speeds.

The thermal cycler for carrying out an automated PCR process is capable of changing the temperature of the air around the chamber for producing labelled target polynucleotide molecule. The thermal cycler also provides the conditions necessary for the binding reaction of the targets onto their capture molecules.

The thermal cycler is preferably composed in its simplest version of the following relevant components: a thermocouple, a transmitter, a converter and a heater.

The thermocouple, sticks as close as possible to the localized area of the micro-array to be heated, measures the temperature through the transmitter. This temperature information is given to a computer via the converter. Every 0.1 second, the software compares the real temperature measured to the temperature set point requested by the final user. If the measured temperature is higher than the requested one, the heater is simply stopped. If the measured temperature is lower than the set point, the system continues the heating process.

The heating and cooling is preferably obtained using pulsed air and the heater is a electric resistance.

Preferably the heating and cooling systems have a temperature accuracy of less than 1°C and more preferably of less than 0.25°C (at constant temperature). Preferably, the uniformity of heat on the surface of the reaction chamber is at least 1°C and preferably at least 0.1°C. Preferably the accuracy of the temperature is less than 0.5°C and preferably less than 0.1°C.

In a preferred embodiment, changing the temperature of the air around the chamber is performed by pulsed air at a ramp rate (heating or cooling) of 5°C per sec and preferably 10°C per sec and even more preferably 20°C per sec. Preferably the cooling is a passive cooling, drawing in ambient air.

Beside the heating system, the detection requires mainly an illumination light source and a detector (9) for measuring a signal from the bound labelled target polynucleotide molecule. Preferably, a light source generates a beam of light to excite the bound labeled targets bound at the flat surface of the reaction chamber. The detection has to be settled in such a way as to obtain the same detection efficiency on the overall surface covered by the micro-array to be analyzed. A typical detector used in this context is a CCD camera capable to take a picture of the whole micro-array.

In a preferred embodiment, the illumination light and the detector are placed in a room which is isolated from the heated rotor room because of high temperature conditions which may be deleterious for the optic parts. Preferably, a window separate the illumination light source from the heated rotor room while interfering as less as possible with the lighting performance (intensity, focus, direction, ...). Preferably, the same window separates the detector from the heated rotor room while interfering as less as possible with the optical performance (energy, focus, direction,...). The window is preferably placed perpendicular to the optical axis. The inner surface of the rotor room is preferably black and non reflective to prevent any illumination disturbance during the reading. Rotor room volume should be kept low to reduce heating and cooling times. The rotor room is also preferably light tight to avoid external illumination sources.

In a preferred embodiment, the excitation light (7) is a (red) laser diode preferably having a wavelength of 635 nm ± 5 nm. The laser diode is preferably *MRL-635* operating at temperature comprised between 10 and 35°C and having an output power of 50 to 500 mW. The laser has preferably a low heat emission component in order to reduce distortion. Preferably, illumination comes from below the rotating holder. The laser beam is also preferably perpendicular (+/- 20 °) to the flat surface having fixed capture molecules. A mirror can be placed between the laser beam and the flat surface to reduce linear beam length.

Preferably, the spectral line width is maximum 5 nm and even 3 mm in order to avoid non-specific excitation and emission overlap. Preferably, the beam homogeneously illuminates the total surface of the micro-array, being preferably a surface of 20 x 10 mm. Homogeneity should be at least 95% over the whole surface and even at least 98%.

Preferably, the laser beam generated by the light source is nearly collimated and nearly Gaussian.
Gaussian profiles for the illumination beam can be obtained by spatial filters or coupling to single mode optical fibers or alternatively by using solid state lasers such as DPSS.

An exchangeable emission filter is used to collect only the wavelengths of interest. An additional filter wheel is preferably placed and used as an attenuation filter to precisely regulate the laser power. This filter wheel is shaded differently at variable know absorption levels. A lens that may be anti-reflection coated is used for focusing the laser beam on the flat surface of the reaction chamber. In a preferred embodiment, the detector (9) comprises an optic lens (10). The distance between the light source, the lens and the flat surface is variable to allow focusing.

Emitted light (8) is focused through an optical lens (10) to a detector (9) for detecting the number of photons present therein. The detector (9) is preferably a cooled CCD camera. The camera is preferably a monochrome CCD (no need for RGB filters). The camera is selected in order to provide maximum Quantum Efficiency at the wavelength of emission which is preferably 670 nm. The CCD quantum efficiency is preferably at least 20 % at 670 nm, preferably higher than 50%. Preferably the CCD pixel size lies between 8 and 24 µm. The CCD sensor area is at least 20 x 10 mm in order to obtain a 1:1 ratio and best image perspective in the tilted CCD setup. The CCD camera is preferably a full frame camera (no Interline sensor with micro-lenses) for reading in the forbidden angle. Image digitization is preferably at least 12 bits in order to obtain sufficient quantification levels and more preferably at least 14 bits. The exposure time is preferably lower than 10 sec and preferably lower than 2sec. The image download time is preferably less than 5 sec and preferably less than 3 sec to allow real-time quantification. Mirrors are preferably avoided to prevent dust accumulation and picture deterioration. The camera is preferably mounted at an observation angle θobin relative to the normal to the said solid support surface in the support, such that 90° > θobin > sin⁻¹ (n2/nl).

The lens (10) has preferably the following features: a large image circle (i.e. a diameter of 43.2mm), a large aperture: f/2,8 going down to f/8 or less, a short focal length (i.e.40 mm), a macro capability of less than 10 cm, is suitable for a 1:1 size ratio.

In a specific embodiment, an emission filter that transmits light having a wavelength greater than about 665 nm is added. The emission filter is preferably placed on the lens or close to the lens. The emission filter has preferably the following features: a low frequency pass combined with a band pass, having for example a cut-on frequency of 665 nm and cut-off at 700 nm, a transmittance of bandwidth higher than 0.8 T and a transmittance outside of bandwidth lower than 0.001 T, a transmittance between 500 and 665 nm lower than 0.0001 T, an accuracy of bandwidth ± 3 nm.

In a preferred embodiment, the illumination light source produces an excitation light (7) which is directly focused on the flat surface of the reaction chamber, wherein the excitation light reaches the micro-array surface within an angle comprised between 45 and 135°. Preferably, the excitation light (7) reaches the surface of the support with an angle of about 90°, thus normal to the surface. Calculated according to the normal, this angle would be about 0°. Thus, the excitation light reaches the flat surface of the reaction chamber within an angle which does not induce internal reflection of light as provided by evanescence wave.

In a preferred embodiment, the detection is performed using an evanescent field as described in the US Patent Application 11/526,159. An "evanescent field" or "evanescent wave" is used herein with its commonly understood meaning, *i*.*e*., refers to an exponentially decaying electromagnetic field generated on the far or distal side of a totally internally reflecting interface that is illuminated by an incident light source. Generally, the excitation energy of the evanescent wave is the same as the energy of the wavelength of the incident light that was totally internally reflected. Typically, the evanescent field propagates with significant energy for only a relatively short distance from the distal surface of the interface (e.g., on the order of magnitude of its wavelength).

Preferably, the illumination is such as to obtain Total Internal reflection fluorescence (TIRF) and homogeneous evanescent field on the surface of the solid support having capture molecules immobilized thereon.

In a preferred embodiment, the excitation light source (1) produces an evanescent field. Preferably, the evanescent field is generated by an incident light source illuminating the surface of the solid support with an incidence angle comprised between about 60° and 90° from the normal.

In another preferred embodiment, the evanescent field excites the label of the labeled amplicons and emitted signal is detected by a detector. Preferably the detector comprises a CCD camera. In another embodiment, the detector comprises a photomultiplier.

In a preferred embodiment, the incident light source, the solid support and the detector are not moving relative to each other during the detection. This is the simplest system. The CCD camera collects the light emitted from the solid support surface in a single acquisition.

In another preferred embodiment, the detector is positioned at an observation angle θobin relative to the normal to the said solid support surface in the support, such that 90° > θobin > sin⁻¹ (n2/n1) as described in the European Patent Application EP07112900.1.

The method for detecting a biological target molecule present on an optically transparent solid support surface having a refractive index n1, said solid support being in contact with a medium having refractive index n2, whereby n1 > n2, said method comprising the steps of: illuminating the target molecule, thereby causing the target molecule to emit light, detecting light emitted from the target molecule through said support at an observation angle θobin relative to the normal to the said solid support surface in the support, such that 90° > θobin > sin⁻¹ (n2/n1).

In a preferred embodiment, the signal is the measured through the support bearing the bound capture molecules at an observation angle θobin relative to the normal to the said solid support surface in the support, such that 90° > θobin > sin⁻¹(n2/n1), whereby the optically transparent solid support having a refractive index n1 and being in contact with a medium having refractive index n2, whereby n1 > n2. Preferably, the observation angle is within the forbidden angle and being in the range of the critical angle plus 10°, preferably plus 5° and more preferably plus 3°.

In another embodiment, the signal is a scattered light from the bound labelled target polynucleotide molecule in response to illumination. In an alternative embodiment, the signal is the result of light diffraction from the bound labelled target polynucleotide molecule in response to illumination.

In a preferred embodiment, the PCR and the detection are performed in a closed cartridge. In another embodiment, the PCR and detection are performed in a same solution.

In a preferred embodiment, the cartridge further comprises a cap for sealing said inlet port, preferably a screwing cap.

In another embodiment, the cartridge further comprises a second reaction chamber said second reaction chamber comprising a channel constructed to permit fluid flow from said inlet port into said second reaction chamber, said channel being connected to the channel constructed to permit fluid flow from said inlet port into the reaction chamber (2) carrying the micro-array.

The cartridge is preferably composed of two connected reaction chambers, one being the reaction chamber comprising the micro-array where the detection is performed and a second chamber. A drawing of a preferred cartridge is presented in figure 9. The second chamber may be used for different purposes. One application is to use the second chamber as a reservoir. The amplification solution may be transferred from the reaction chamber to this second chamber, thus allowing a reading of the micro-array in absence of solution. The second reaction chamber may comprise a flat surface or may be of a different shape (tube, well, etc.). Another application would be to use the second reaction chamber to perform the PCR cycle of denaturation, annealing and elongation and then to transfer the solution to the reaction chamber for hybridization and detection (in presence or not of the solution).

The cartridge is preferably placed and locked on the rotating holder (1) or rotor of the apparatus with the inlet port oriented upside and with their length axis on a diameter of the rotor. The invention will now be described in detail by means of the following non-limiting examples with reference to the enclosed figures.

### Example 1. Multiplex PCR efficiency in flat surface reaction chamber or performed in PCR tubes and hybridization of the amplicons on microarray

A reaction chamber having a large flat surface made in Zeonex has been used to make PCR amplification. The plastic device was made of 2 plastic parts: the first part was in black Zeonex and had dimensions of 40 mm X 25 mm X 3 mm including a cavity of 20 mm X 20 mm X 400 µm. The second part was a transparent Zeonex cover having dimensions of 40 mm X 25 mm X 1 mm or 0.5 mm in thickness. The 2 plastic parts were sealed together by laser welding to form a closed chamber with dimensions of 20 mm X 20 mm X 400 um. The chamber contains 2 silicone plugs of 1 mm diameter each one inserted in an inlet to inject the liquid into the chamber using a syringe.

The multiplex PCR amplification was performed using the following protocol.

The PCR solution contained: 1X Buffer Biotools including 2 mM MgCl₂, potassium glutamate 40 mM (Sigma, St Louis, USA) and 3.5% of Dextran D1662 (Sigma, St Louis, USA), a primer mix of the DualChips GMO (Eppendorf, Hamburg, Germany) containing 13 primer pairs at 50 nM each with one of the primer being biotinylated at 5' end, 200 µM of each dATP, dCTP, dGTP , 100 µM of dTTP and 300 µM of dUTP, 2.5 U/25µl of Taq DNA polymerase (ref. 201203, Qiagen, Hilden, Germany), 0.5 U/25µl of UNG (ref 71960, USB, Cleveland, Ohio, USA) and containing 100 ng/25 µl of a DNA mix made of Genomic DNAs that were extracted from different samples : using a CTAB-based method (Rogers and Bendich, 1985) and quantified using the "Quant-it™ PicoGreen dsDNA assay kit" (Invitrogen, USA) as described in the manual.
The 100 ng target DNA mix was made of:
BT11 0,1 % (ERM-BF412f, IRMM, Geel , Belgium)
RRS 0, 1 % (ERM-BF410f, IRMM, Geel , Belgium)
Ga21 0,1 % (ERM-BF414f, IRMM, Geel , Belgium)
Mon 810 0,1% (ERM-BF413f, IRMM, Geel , Belgium)
BT176 0,1% (ERM-BF411f, IRMM, Geel, Belgium)
GT73 0,1% (AOCS 0304-B, Urbana, IL, USA)
The target DNA mix contained the following 12 genetic elements: P35S, T-nos, Pat, Cry1Ab-1, CrylAb-2, Cry1Ab-3, EPSPS-1, EPSPS-2, Invertase (Maize), Lectin (Soybean), Cruciferin (Rapeseed) and Rbc1 (Plant universal).
The multiplex PCR contained primer pairs for amplification of 13 genetic elements of GMOs as in the kit DualChips GMO (Eppendorf AG, Hamburg, Germany). The 13 genetic elements are the following: P35S, T-nos, Pnos-nptII, Pat, Cry1Ab-1, Cry1Ab-2, Cry1Ab-3, EPSPS-1, EPSPS-2, Invertase (Maize), Lectin (Soybean), Cruciferin (Rapeseed) and Rbc1 (Plant universal).
PCR mix samples have been injected through silicone balls into a 200 µl flat surface plastic chamber covered with plastic cover of 1 mm thickness or 500 µm thickness. The same solution (25µl) was added to a normal 250 µl polypropylene PCR tube (Eppendorf AG, Germany) having walls 250 µm thick. Plastic chambers were attached with silicone string onto a rotor of an air heating PCR cycler Rotor-Gene 6000 (Corbett Life Science, Sydney, Australia). The PCR tubes were also incorporated onto the rotor holes dedicated for PCR tubes. All the holes of the rotor were filled with empty PCR tubes. Rotor started to rotate at 400 rpm and the protocol of PCR was the following: samples were first incubated at 22°C for 10 min in order to allow the UNG action and then denatured at 94 °C for 3 min. Then 40 cycles of amplification were performed consisting of 30 sec at 94 °C, 90sec at 56 °C and 30 sec at 72 °C and a final extension step of 10 min at 72 °C.

### Hybridisation

The hybridization of the amplicons was performed on DualChips GMO covered with hybridization frames as recommended by the manufacturer (Eppendorf AG, Hamburg, Germany). 25 µl of solution containing amplicons were transferred onto a DualChip GMO array inside a hybridization chamber without any addition of solution or reagent. The hybridisation was carried out at 60° for 1 h. Slides were washed and detected as described in the DualChip GMO kit using the Silverquant detection kit ( Eppendorf, Hamburg, Germany). The slides were dried and analysed using a microarray reader. Each array (slide) was then quantified by the DualChip GMO evaluation tool (Eppendorf, Hamburg, Germany). Figure 6 shows the quantification of the signal of 12 GMO genetic elements and the comparison between the detection efficiency between a flat surface device PCR amplification having a thickness of 0.5 mm or 1 mm and PCR tube amplification.

### Example 2. Comparison between PCR amplification in a reaction chamber having a flat surface or in a PCR tube in a cycling device based on a Peltier heating block or on hot air

The experiment was performed as described in example 1 except that the target DNA was a plasmid (reference) corresponding to P35 genetic element of the GMO and the enzyme used was the Qiagen Hotstart Taq polymerase (ref. 203203, Qiagen, Hilden, Germany). The expected size for the amplicon was 73 bp.
100 µl of PCR reaction have been introduced either in flat plastic devices with a cover of 1 mm thick or 500 µm thick or in a PCR tubes having a wall thickness of 250 µm.
One flat plastic device with thickness of 500 µm and one device with a thickness of 1 mm have been fixed into a Corbett Cycler Rotor-Gene 6000 (Corbett Life Science, Sydney, Australia) as shown in figure 1. The PCR tubes were inserted into Master cycler (Eppendorf, Hamburg, Germany) together with the flat reaction chambers which were placed on a thermic adapter in order for the flat surface to be into contact with the metal. The flat reaction chambers are positioned into the thermic adaptor and then the adaptor is inserted into a 96-wells thermoblock of the PCR thermocycler.
The PCR protocol was the following: samples were first incubated at 25°C for 10 min and then denatured at 94 °C for 15 min. Then 30 cycles of amplification were performed consisting of 30 sec at 94 °C, 90 sec at 56 °C and 30 sec at 72 °C and a final extension step of 10 min at 72 °C.
The appearance of the flat reaction chamber when PCR was performed in a Hot air cycler (Corbett cycler) was a smooth surface covered homogeneously with water. In contrast, the surface of the flat reaction chamber was covered with small air bubbles when the PCR was performed in a Peltier cycler.
After PCR, 10 µl of PCR solution were loaded onto 2% agarose gel stained with Ethidium bromide and separated by electrophoresis for 30 min at 100 volt. The mass ladder used was the Promega 100 bp DNA ladder G2101. The presence of the high salt leads to the appearance of a broad band.
The band intensity corresponding to the amplified DNA was quantified for each condition and the result is summarized in the table hereafter.

| Condition | Device | Thickness (mm) | Thermocycler | Band intensity | Efficiency of PCR compared to PCR Tube (%) |
|---|---|---|---|---|---|
| 1 | Flat | 0.5 | Peltier block | 5 | 20 |
| 2 | Flat | 1 | Peltier block | 0 | 0 |
| 3 | Flat | 0.5 | Hot air | 18 | 72 |
| 4 | Flat | 1 | Hot air | 6 | 24 |
| 5 | PCR tube | 0.25 | Peltier block | 25 | 100 |

This result shows the evidence of better efficiency of PCR on a flat surface device in hot air cycler compared to the Peltier cycler. The results showed that with a thickness of 500 µm, the PCR performed in a hot air cycler (condition 3) is nearly as efficient (72%) than in a thin PCR tubes (condition 5) in the Peltier cycler used here as a reference ( Mastercycler, Eppendorf, Hamburg). However the presence of a thicker wall of 1 mm reduces the efficiency of the PCR even in a hot air cycler (condition 4). The PCR on a flat surface device in a Peltier cycler is inefficient with a thickness of 500 µm (condition 1) and null with thickness of 1 mm (condition 2).

### Example 3. Multiplex PCR with fluorescent primers and hybridization in the same closed reaction chamber having a microarray on flat surface

The reaction chamber that has been used to perform the PCR and hybridization was a well of a 8-well strip in Zeonex (Eppendorf, Hamburg, Germany) coated with an epoxy layer following the method of the European Patent EP1200204B1. A plasma polymerisation of glycidyl methacrylate was applied to the 8-well strips.
The dimensions of the 8-well strip were the following: 8.07 mm (W) x 82 mm (L) x 4.9 mm (H).
The strip comprises 8 wells and bottom surface of each well was about 40 mm². The thickness of the bottom surface of the wells was 0.9 mm.

The following capture probes sequences were used for the array.

| Name | Sequence 5' to 3' |
|---|---|
| TP35S | GTCATCCCTTACGTCAGTGGAGATAT |
| Tnos | CCGCTTGGGTGGAGAGGCTATTC |
| Tpat | CTGTGTATCCCAAAGCCTCATGCAA |
| Tcry1Ab | CAGACGGTGGCTGAAGCCCTGTCG |
| Tinvertase | TTAGACGGGAAAACGAGAGGAAGC |
| Tcruciferin | TTCAGAGTGCTGATGTAACCGAGCT |
| Trbcl | ATAAGCAATATATTGATTTTCTTCTCCAGCAACGGGCTCGATGTGGTAG CATCGC |

The capture probes sequences comprise a common spacer at their 5' end as provided in the European patent application EP01788098 as SEQ ID NO: 3. The spacer was terminated by an amino group to allow the covalent fixated of the probes into the epoxy coated wells. The capture probes were spotted in duplicate at 3 µM at the bottom of the epoxy coated 8-well strip.
The PCR was performed as in example 1 except that six 5' Cy5 labeled primers at 75 nM were used instead of the biotinylated primer at 50 nM. Their target genes were P35S, T-nos, Pat, Cry1Ab, invertase, and Rbc1. The other 7 primers pairs were unchanged. An engineered Taq polymerase having five DNA binding domains was used and the PCR solution contained glutamate 120 mM.
In this PCR mix, we added 100 ng of BT11 0.1% target DNA (ERM-BF412f, IRMM, Geel, Belgium). The target DNA contained the following 6 genetic elements: P35S, T-nos, Pat, Cry1Ab, Invertase (Maize) and Rbc1 (Plant universal). The array comprises an additional capture probe (Cruciferin) which is used as negative control for the PCR as this element is not present in BT11.
100 µl of PCR mix was introduced into a well of a 8-well strip in Zeonex (Eppendorf) coated with an epoxy layer (following P2I patent) on which the GMO array has been spotted. The wells were closed with aluminium PCR foil (Eppendorf, Hamburg, Germany) and processed in PCR in a Corbett cycler Rotor-Gene 6000. Rotor started to rotate at 400 rpm and the protocol of PCR was the following: samples were first incubated at 25°C for 20 min in order to allow the UNG action and then denatured at 95 °C for 15 min. Then 40 cycles of amplification were performed consisting of 60 sec at 95 °C, 90 sec at 56 °C and 60 sec at 72 °C, then 15 sec at 95°C followed by 15 min at 56°C for hybridization step.
After hybridization step in the Corbett cycler, the wells were removed from the machine and washed. All buffers used were from the Dualchip GMO kit (Eppendorf, Hamburg, Germany).
The wells were washed once for 1 min with 100 µl of Washing Buffer, twice for 1 min with Post hybridization buffer, twice for 1 min with Washing Buffer and finally twice for 1 min with Washing Buffer without Tween. Wells were dried and read in Axon scanner (4100 personal). Scanning was performed with the channel Cy5 at a gain of 900 with a resolution of 10 micrometer.
Figure 7 shows a quantification of the signal for the 6 GMO genetic elements contained in BT11 (P35S, T-nos, Pat, Cry1Ab, Invertase, Rbc1) and the absence of signal on Cruciferin (negative control).

### Example 4. Multiplex real-time PCR on microarray

The experiment is conducted as described in example 3.
A Plastic device in Zeonex is used to perform real-time PCR amplification on array. A picture of the device is provided in figure 9. The plastic device is made of 3 plastic parts assembled together:
(1) transparent part in Zeonex (39 mm X 24 mm X 1 mm) having one cavity (20 mm X 10 mm X 500 µm) at the bottom, a central injection port and two cavities on the top (20 mm x 10 mm x 500 µm and 20 mm x 10 mm x 1 mm),
(2) black part in Zeonex (40 mm X 26 mm X 500 µm) having one hole (20 mm X 10 mm X 500 µm) and
(3) an optic bloc in transparent Zeonex of 24 mm X 14 mm X 3.5 mm in thickness.
A DualChip GMO microarray was spotted on the flat surface of the optical bloc. The chemistry used for the spotting of aminated capture molecules on Zeonex is an epoxide coating (P2i). After the spotting, the 3 plastic parts are welded together, and two reaction chambers are formed: one with dimensions of 20 mm X 10 mm X 500 µm (array chamber) and the other with 20 mm X 10 mm X 500 µm (PCR chamber). The device is sealable with a screw cap which is adapted to fit with the injection port.
Measurement of the labelled target polynucleotide molecules bound to their respective capture molecule is monitored during the PCR cycles.
PCR mix sample is injected through the injection port into the device. The device is closed by the screw cap and is fixed on the rotor holder of the real-time PCR machine with the array chamber positioned in periphery of the rotor holder as provided in figure 8. The apparatus for real time PCR on microarray comprises a hot air thermal cycler having a rotor and a detector which is positioned at an observation angle θobin relative to the normal to the said solid support surface in the support, such that 90° > θobin > sin⁻¹ (n2/n1) as described in the European Patent Application EP07112900.1.
The rotor rotates at 1000 rpm and the protocol of PCR starts as followed:
Samples are first incubated at 25°C for 10 min and then denatured at 95 °C for 15 min. Then 40 cycles of amplification are performed consisting of 60 sec at 95 °C, 90 sec at 56 °C and 60 sec at 72 °C and a final extension step of 10 min at 72 °C.

Excitation of the polynucleotide molecules is done by the use of a red diode laser (MRL-635, Dragonlasers, JiLin, China) illuminating the array directly (from a normal direction or 0°) or with an incidence comprised between 0° and 20°. The fluorescent light emission is determined by collecting the fluorescent signal on the micro-array surface with a CCD camera (ML6303E-2, EHD, Damme, Germany) starting 1 min after the beginning of the annealing step (at 56°C) for 30 sec acquisition of the cycles 6, 10, 14, 18, 19, 20, 21, 22, 23, 24, 25, 26, 27, 28, 29, 30, 32, 34, 36, 38 and 40. The rotor is stopped during the acquisition. In order to avoid detecting the excitation signal, a filter (Cy5 filter set 41008, Chroma, Fuerstenfeldbruck, Germany) is placed between the array and CCD camera. An optical lens (Makro Apo-Componon 2.8/40, Bad Kreuznach, Germany) is also placed between the polynucleotides and the camera, allowing a mangification ratio of 1 to 1 when placed at 80mm from the array. Generally, the filter is placed just in front of the lens, filtering light coming from the array before it enters the optical lens.

## Claims

**1.** A method for PCR amplification and detection of a polynucleotide molecule being present in a solution contained in a chamber having a surface bearing the capture molecules for the detection of the amplified sequences comprising the steps of :
- providing a rotating holder (1) and a reaction chamber (2) having fixed upon one of its surface at least a capture molecule (20) being immobilized in a localized areas (21) of a flat surface of said reaction chamber,
- introducing a solution containing said polynucleotide molecule into said reaction chamber (2) and reagents for polynucleotide molecule amplification and labelling,
- submitting the solution to at least 2 thermal cycles having at least 2 and preferably 3 different temperature steps in order to obtain labelled target polynucleotide molecule by PCR amplification, wherein the change in temperatures is obtained by changing the temperature of the air around the reaction chamber (2) and wherein the reaction chamber (2) is subjected to a rotating movement,
- performing at least a measurement of the labelled target polynucleotide molecule in the following way,
• incubating said labelled target polynucleotide molecule present in said solution under conditions allowing a specific hybridization between said target polynucleotide molecule and its corresponding capture molecule (20),
• measuring a signal originating from the surface having the bound labelled target polynucleotide molecule in response to illumination of said flat surface, wherein the signal is measured outside the chamber, and wherein the surface of signal emission comprises at least one localized area having a surface of between about 0.1 µm² and about 75 mm²,
wherein the said surface has a homogeneous interface during the measurement of the signal and
- Processing the data obtained in order to detect and/or quantify the amount of polynucleotide molecule present in the solution before the amplification.

**2.** The method of claim 1,
wherein the measurement of the labelled target polynucleotide molecule is performed with the reaction chamber being on said rotating holder; or
wherein the measurement of the labelled target polynucleotide molecule is performed at least once during the PCR amplification; or
wherein the surface bearing the bound capture molecules has a homogeneous interface after a PCR cycle; or
wherein the interface between the said surface containing bound target and the solution is homogeneous in at least 90% of the surface; or
wherein the height of the liquid in the detection part of the chamber is preferably comprised between 0.1 and 5 mm, and even more preferably between 0.2 and 2 mm; or
wherein the signal is the result of light emission (8) from the bound labelled target polynucleotide molecule in response to excitation light (7).

**2.** The method of claim 1, wherein the measurement of the labelled target polynucleotide molecule is performed in presence of the amplification solution containing the labelled target polynucleotide molecules.

**3.** The method of claim 2,
wherein the surface of the reaction chamber having fixed capture molecule is transparent to the excitation and/or emission light; or
wherein at least one side and/or the top of the reaction chamber is in a material transparent to the excitation and/or emission light; or
wherein light emission is due to evanescence excitation; or
wherein the detected signal is light due to evanescence emission; or
wherein the excitation light is totally internally reflected inside the transparent support; or
wherein the detected emitted light is totally internally reflected inside the transparent support; or
wherein the excitation light is the result of a light beam focused on the surface of the chamber having bound capture molecules.

**4.** The method of claim 1, wherein the detected signal is the measured through the support bearing the bound capture molecules at an observation angle θobin relative to the normal to the said solid support surface in the support, such that 90° > θobin > sin⁻¹ (n2/nl), whereby the optically transparent solid support having a refractive index n1 and being in contact with a medium having refractive index n2, whereby n1 > n2.

**5.** The method of claim 4, wherein, the observation angle is within the forbidden angle and being in the range of the critical angle plus 10°, preferably plus 5° and more preferably plus 3°.

**6.** The method of claim 1,
wherein the signal is a scattered light from the bound labelled target polynucleotide molecule in response to illumination; or
wherein the signal is the result of light diffraction from the bound labelled target polynucleotide molecule in response to illumination; or
wherein the PCR and the detection are performed in a closed cartridge; or
wherein the PCR is performed with the support being rotated at a speed of at least 100 and preferably 400 and still preferably 1000 rpm; or
wherein the measurement of the labelled target polynucleotide molecule is performed when the chamber is subjected to a rotating movement; or
wherein the measurement of the labelled target polynucleotide molecule is performed when the chamber is not subjected to a rotating movement; or
wherein the measurement of the labelled target polynucleotide molecule is performed in at least 5, preferably at least 10 thermal cycles and even preferably at least 20 thermal cycles; or
wherein subjecting the reaction chamber to a rotating movement during the thermal cycles prevents a presence of bubbles on the localized areas.

**7.** The method of claim 1, wherein the solution containing the labelled target polynucleotide molecules is moved from the reaction chamber to a second reaction chamber by centrifugation.

**8.** The method of claim 7, wherein reading of the bound labelled target polynucleotide molecules is performed in the reaction chamber in absence of solution comprising the labelled target polynucleotide molecules.

**9.** The method of claim 1, wherein the reagents for polynucleotide molecule amplification comprise a primer pair, dNTPs, a thermostable DNA polymerase, a hot start PCR system and buffer.

**10.** The method of claim 9, further comprising a salt composition having at least 100 and preferably 150 mM and even more preferably 200mM of cations.

**11.** The method of claim 9, further comprising a salt composed of a cation and an anion,
wherein the said anion has two carboxylic groups and one amine group, wherein the salt concentration in the composition is comprised between 10 mM and 400 mM and from 1% to 20% by weight of an exclusion agent.

**12.** The method of claim 11, wherein the anion is glutamate.

**13.** The method of claim 1,
wherein the steps of denaturation, annealing, elongation are performed in 1 min or less; or
wherein the Tm of the primers(Tmp) for a target are within a range of the temperature of annealing (Ta) -2 to +8°C and preferably 0 to +4°C; or
wherein the Tm of the capture molecule for a target is within a range of temperature of the hybridization + 4 to 16°C preferably + 8 to 12°C; or
wherein the Tm of the two capture molecules differing from one base and use for the discrimination of a SNP in a target sequence have a Tm within a range of temperature of hybridization plus 4 to 8°C; or
wherein the Tm of the primer is at least 4°C and preferably 8°C lower than the Tm of the capture molecule; or
wherein the capture molecule has a spacer of at least 6.8nm long being preferably a sequence of at least 20 nucleotides and preferably more than 40 nucleotides long; or
wherein the hybridization and the annealing are performed in the same step.

**14.** A method for PCR amplification and detection of a polynucleotide molecule being present in a solution contained in a chamber having a surface bearing the capture molecules for the detection of the amplified sequences comprising the steps of :
- providing a rotating holder (1) and a reaction chamber (2) having fixed upon one of its surface at least a capture molecule (20) being immobilized in a localized areas (21) of a flat surface of said reaction chamber,
- introducing a solution containing said polynucleotide molecule into said reaction chamber (2) and reagents for polynucleotide molecule amplification and labelling,
- submitting the solution to at least 2 thermal cycles comprising a denaturation, annealing and elongation steps in order to obtain labelled target polynucleotide molecule by PCR amplification,
- performing at least a measurement of the labelled target polynucleotide molecule in the following way,
• incubating said labelled target polynucleotide molecule present in said solution under conditions allowing a specific hybridization between said target polynucleotide molecule and its corresponding capture molecule (20),
• measuring a signal originating from the surface having the bound labelled target polynucleotide molecule in response to illumination of said flat surface, wherein the signal is measured outside the chamber, and wherein the surface of signal emission comprises at least one localized area having a surface of between about 0.1 µm² and about 75 mm²,
wherein the said surface has a homogeneous interface during the measurement of the signal,
• wherein the hybridization and the annealing steps occur at the same temperature and wherein the Tm of the primers for a target are within a range of the temperature of annealing plus 0-4°C and the Tm of the probe for said target is within a range of temperature of the hybridization plus 6-12°C and
- Processing the data obtained in order to detect and/or quantify the amount of polynucleotide molecule present in the solution before the amplification.

**15.** The method of claim 1 and 14,
wherein the hybridization, annealing and elongation are performed in the same step; or
wherein the steps of annealing and hybridization are performed in 2 min or less; or
wherein the Tm of the capture molecule for a target is at least 4°C and preferably 6°C higher than the Tm of the two primers specific of said target; or
wherein all the capture molecules and primers of the targets to be detected are within the range given in claim 33 to 35.

**16.** The method of claim 1,
wherein the reagents for polynucleotide molecule amplification comprise a primer and/or dNTP labelled with a fluorescent dye; or
wherein the flat surface is at least 0.04 cm² or preferably at least 1 cm² or even more preferably more than 2 cm²; or
wherein the flat surface is at least 0.25 mm thick and preferably 0.5 mm thick; or
wherein at least one part of the device is a conductive material; or
wherein the flatness of the surface is changed by less than 0.05 mm after at least 20 amplification cycles, or
wherein the localized area is comprised between about 10 µm² and about 1 mm² and preferably between about 1 µm² and about 100 µm².

**17.** The method of claim 1, wherein the capture molecules are bound to a localized area of the flat surface in the form of a micro-array.

**18.** The method of claim 17, wherein the micro-array comprises more than 5 different capture molecules (20), preferably more than 20 and even more than 50.

**19.** The method of claim 1,
wherein the rotating holder (1) bears several micro-arrays separated by physical boundaries; or
wherein the rotating holder (1) has a disk shape.

**20.** The method of claim 1, wherein the rotating holder (1) has a multi-well plate or strip format.

**21.** The method of claim 20, wherein the multi-well plate is submitted to a temperature gradient during the measurement of light emission.

**22.** The method of claim 1,
wherein the light emission (8) is measured at a defined timing from the beginning of a temperature step; or
wherein the light emission (8) is measured at within 5 min and even within 2 min and more preferably within 1 min after the beginning of the annealing temperature step; or
wherein the light emission (8) is measured at the end of at least one of the 3 temperature steps used for the PCR amplification; or
wherein the light emission (8) is measured at the end of the PCR amplification; or
wherein the data are processed in order to obtain a signal value for each of the localized area; or
wherein the data are processed in order to obtain a signal value for each of the localized area and for the local background, or
wherein the data are further processed by subtracting the background from the signal value for each of the localized area; or
wherein the quantification of the amount of polynucleotide molecule is performed by comparing the signal value of the localized area with a fixed value.

**23.** The method of claim 1, wherein the quantification of the amount of polynucleotide molecule is performed by comparing the number of thermal cycles necessary to reach a fixed value (CT) with the CT of a reference polynucleotide molecule.

**24.** The method of claim 23,
wherein the reference polynucleotide molecule is amplified in the same solution and detected on the same micro-array as the target polynucleotide molecule; or
wherein the polynucleotide molecule is labelled with a first fluorescent dye and the reference polynucleotide molecule is labelled with a second fluorescent dye different from the first fluorescent dye.

**25.** The method of claim 1,
wherein the quantification of the amount of polynucleotide molecule is performed by comparing the number of thermal cycles necessary to reach a fixed value (CT) with a standard curve wherein the CT are plotted against standard concentrations; or
wherein two fluorescent dyes are used in the same solution; or
wherein the solution composition is adapted for performing the annealing of the primers on the polynucleotide molecule and the hybridization of the labelled target molecule on the capture molecule during the same temperature step; or
wherein the capture molecules (20) bound to the labelled target polynucleotide molecules are elongated during the temperature step of elongation; or
wherein the capture molecules elongated are detected during the temperature step of denaturation; or
wherein, the reagents for polynucleotide molecule amplification comprises at least 5 primer pairs, preferably at least 10 primer pairs, more preferably at least 20 primer pairs et even at least 40 primer pairs; or
wherein between 1 and 4 polynucleotide molecules and preferably between 1 and 20 polynucleotide molecules present in a solution are amplified and detected and/or quantified in the same assay, or
wherein between 20 and 1000 polynucleotide molecules present in a solution are amplified and detected and/or quantified in the same assay; or
wherein an excitation light (7) from a light source is directed on the surface of the support; or
wherein the detected light is the light emitted by the bound target molecule under excitation from a light source; or
wherein a thermal cycle is performed within 10 min and preferably within 3 min and even more preferably within 2 min, or
wherein 30 thermal cycles are performed within 5 h and preferably within 3 h and even more preferably within 1.5 h; or
wherein the capture molecule is a single stranded polynucleotide containing a sequence able to specifically bind the labelled target polynucleotide molecule and a spacer of at least 20 nucleotides; or
wherein changing the temperature of the air around the chamber is obtained by pulsed air.

**26.** An apparatus for monitoring on a micro-array a PCR amplification of a polynucleotide molecule being present in a solution comprising:
- A rotating holder (1) and a reaction chamber (2) having fixed upon its surface a micro-array, comprising at least one capture molecule (20) being immobilized in specifically localized areas (21) of a flat surface of said chamber, which is in fluid communication in a chamber with said polynucleotide molecule and reagents for polynucleotide molecule amplification and labelling,
- a thermal cycler for carrying out an automated PCR process, said thermal cycler capable of changing the temperature of the air around the chamber for producing labelled target polynucleotide molecule,
- a rotor for rotating said holder (1) and said reaction chamber (2),
- an illumination light source,
- a detector (9) for measuring a signal from the bound labelled target polynucleotide molecule, with said solution being present in the chamber and containing the labelled target polynucleotide molecule wherein the surface of emission for a localized area is comprised between about 0.1 µm² and about 75 mm² and has a homogeneous interface,
wherein the different parts are integrated into the same apparatus in order to read the signal of the bound labelled target polynucleotide molecule during the PCR amplification.

**27.** The apparatus of claim 26,
further comprising:
- a storage system for storing the data of the different measurements for at least 5 localized areas of the support at a defined timing of a thermal cycle,
- a controller repeating the steps of illumination, detection and storage at least one time in at least one thermal cycle for each localized area of the micro-array,
- a program for processing the data obtained in at least one thermal cycle in order to detect and/or quantify the amount of polynucleotide molecule present in the sample before the amplification; or
wherein the rotating holder (1) has a disk shape; or
wherein the rotating holder (1) is the rotor; or
wherein the rotating holder (1) comprises a plurality of reaction chambers (2); or
wherein the detector (9) comprises an optic lens (10), or
wherein the micro-array comprises more than 5 different capture molecules (20), preferably more than 20 and even more than 50; or
wherein changing the temperature of the air around the chamber is performed by pulsed air at a ramp rate of 5°C per sec, or
wherein the localized area is comprised between about 10 µm² and about 1 mm² and preferably between about 1 µm² and about 100 µm²; or
wherein said illumination light source produces an excitation light (7) which is directly focused on the flat surface of the reaction chamber, wherein the excitation light reaches the micro-array surface within an angle comprised between 45 and 135°; or
wherein the illumination light source produces an evanescent field; or
wherein the evanescent field is generated by an incident light source illuminating the surface of the reaction chamber with an incidence angle comprised between about 60° and 90°; or
wherein the detector comprises a CCD camera.

**28.** The apparatus of claim 26, wherein the detector is positioned at an observation angle θobin relative to the normal to the said flat surface of the reaction chamber, such that 90° > θobin > sin⁻¹ (n2/n1).

**29.** The apparatus of claim 28, wherein the observation angle is within the forbidden angle and being in the range of the critical angle plus 10°, preferably plus 5° and more preferably plus 3°.

**30.** A cartridge for monitoring on a micro-array a PCR amplification of a polynucleotide molecule being present in a solution, said cartridge comprising:
- a substrate (optic bloc) comprising a first flat surface and a flat second surface, said first flat surface comprising a micro-array comprising at least 20 different capture molecules (20) being immobilized in specifically localized areas (21); and
- an airlock comprising an inlet port, a mounting surface and a reaction chamber (2); said reaction chamber comprising a channel constructed to permit fluid flow from said inlet port into said reaction chamber (2), wherein said first flat surface of said substrate is mounted with respect to said mounting surface thereby covering said reaction chamber and whereby said micro-array is located inside said reaction chamber (2).

**31.** The cartridge of claim 30,
further comprising a cap for sealing said inlet port, preferably a screwing cap; or
further comprising: a second reaction chamber said second reaction chamber comprising a channel constructed to permit fluid flow from said inlet port into said second reaction chamber, said channel being connected to the channel constructed to permit fluid flow from said inlet port into said reaction chamber (2).

**32.** A kit for monitoring on a micro-array a PCR amplification of a polynucleotide molecule being present in a solution, said kit comprising a cartridge having a substrate (optic bloc) comprising a first flat surface and a flat second surface, said first flat surface comprising a micro-array comprising at least 20 different capture molecules (20) being immobilized in specifically localized areas (21) and primers for the amplification of target, wherein the Tm of the immobilized probes for said target is at least 4°C and preferably 6°C higher than the Tm of the two primers specific of said target and a solution for the PCR including glutamate.
